(19) Europäisches Patentamt
European Patent Office
Office européen des brevets

(11) EP 4 567 050 A1

# (12) EUROPEAN PATENT APPLICATION

(43) Date of publication:
**11.06.2025 Bulletin 2025/24**

(21) Application number: **24209729.3**

(22) Date of filing: **30.10.2024**

(51) International Patent Classification (IPC):
***C08B 37/08*** (2006.01) ***A61K 8/73*** (2006.01)
***C08J 3/075*** (2006.01) ***C08J 3/24*** (2006.01)

(52) Cooperative Patent Classification (CPC):
**C08B 37/0072; A61K 8/735; C08J 3/075; C08J 3/24;** C08J 2305/08

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.12.2023 TR 202316429**

(71) Applicant: **Quantum Beauty Cosmetics Joint Stock Company**
**34746 Atasehir, Istanbul (TR)**

(72) Inventor: **Gholami, Hoshyar**
**Beyoglu (TR)**

(74) Representative: **Erbacher, Martin**
**Boehmert & Boehmert**
**Anwaltspartnerschaft mbB**
**Pettenkoferstrasse 22**
**80336 München (DE)**

## (54) A METHOD FOR PREPARING A CROSS-LINKED HYALURONIC ACID GEL

(57) The present invention provides a method for preparing a cross-linked hyaluronic acid gel comprising the steps:
- cross-linking hyaluronic acid with a cross-linking agent to obtain cross-linked hyaluronic acid;
- adding the cross-linked hyaluronic acid to an alcohol to obtain a first reaction mixture;
-adding the first reaction mixture to an alkaline alcoholic solution to obtain a second reaction mixture;
- separating at least one sediment from the first reaction mixture and/or at least one sediment from the second reaction mixture;
- treating the combined sediments with a buffer having a phosphate buffer to obtain the cross-linked hyaluronic acid gel.

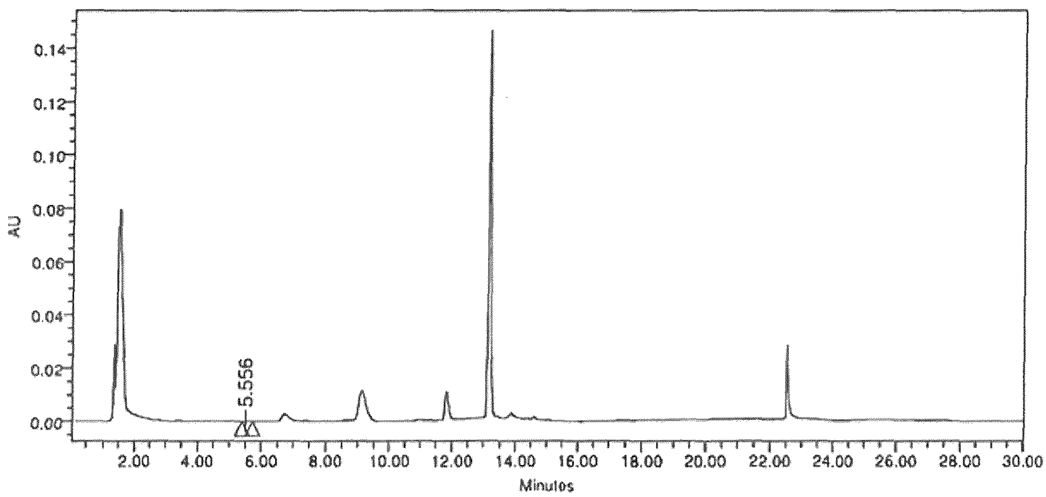


Peak Results

| | Name | RT | Area | Height | Amount | Units |
|---|---|---|---|---|---|---|
| 1 | Blank | 5.556 | 258 | 35 | N.A | N.A |

Fig. 1

EP 4 567 050 A1

## Description

TECHNICAL FIELD

The present invention relates to a method for preparing a cross-linked hyaluronic acid gel. Especially, the present invention relates to reducing the amount of bonding agent consumption, time and cost, as well as increasing efficiency, by preparing stable hyaluronic acid cross-linked powder with a new method for the preparation of various gels.

BACKGROUND

Hydrogels are cross-linked hydrophilic polymers that have the ability to absorb and store large amounts of aqueous solutions. The hydrophilic nature of hydrogels is due to the presence of hydrophilic groups (such as COOH, -OH and $-CONH_2$) in the chain structure of these polymers. [1-4]

Although it has not been half a century since their commercialization, these polymers have found a suitable position in terms of diversity in consumption. Hydrogels, like all engineering materials, must have properties suitable for their applications in order to show the best performance.

Factors that have a significant impact on the properties and applications of hydrogels include solubility, low cost of raw materials and production process, stability, high durability and biodegradability. [5-7]

However, it is not possible in practice to obtain a hydrogel that has all of these properties at the same time. In fact, the nature of the structure, chemical factors and factors acting in the production process are such that the improvement of some properties leads to the loss of some other properties (for example, with a set of fixed raw materials, any composition that increases absorption reduces the strength of the swollen gel).

Therefore, the formulation and process conditions must be designed and optimized to ensure that the produced hydrogel has an appropriate balance of properties depending on the intended application.

One of the methods of changing the properties of hydrogels is to create transverse links between polymer chains. Radiation, physical or chemical methods are used for this work, and as a result, a three-dimensional network is created. [8-9]

The created network must be resistant to heat and mechanical stress, and on average each chain must interact with more than two chains. Chemical methods that create covalent bonds between chains are more useful than radiation methods due to their stability and controllability. [10-14]

The choice of material type is also one of the factors affecting achieving the right hydrogel for the intended application. For example, in medical applications, choosing the materials that are most compatible with the body and do not cause side effects is a sensitive issue. Among these materials, biopolymers such as hyaluronic acid (HA), collagen, alginate and chitosan show high compatibility, and on the other hand, they also contain functional groups such as hydroxyl and carboxylic acid, which inherently have chemical reaction potential.[15-18]

Collagen is used as a natural polymer in the medical field. In the early 1980s, the first collagen-based dermal filler product obtained from cow eye vitreous was launched by Allergan under the brand ZYDERM. However, since these types of fillers are of animal origin, immune and allergic reactions in the human body have limited their use. Accordingly, animal collagen fillers have lost their demand worldwide. As for hyaluronic acids, the first marketed HA-based dermal filler was also of animal origin and was called Hylaform® (Hylan B Gel). This product was discontinued due to problems arising from protein deficiency (pure protein deficiency) and was replaced by bacterial HA-based dermal fillers, known as the most dominant product on the market to date.[19]

HA is found in all vertebrates as a major component of extracellular matrices (ECMs) in most adult tissues. This macromolecule is found in the vitreous of the eye (0.1-0.4 mg/g wet weight in humans), in the synovial joint fluid (3-4 mg/ml), in the matrix produced by cumulus cells around the oocyte before ovulation (0.5 mg/ml), and in other organs. It is an important substance.[20]

The largest amount of HA (7 to 8 grams per average adult human, 50% of the total body) is found in skin tissue. So much so that it is found both in the middle layer of the skin (dermis) (0.5 mg/g wet tissue) and in the skin (epidermis) (0.1 mg/g wet tissue).[21]

The main function of HA in all tissues containing this natural polymer is to absorb moisture and lubricate the tissue. Due to the high concentration of HA in the skin and its elasticity, as age progresses and the half-life of this substance shortens (less than 24 hours in the skin and less than 12 hours in the blood), it causes more severe symptoms than in other tissues.[22]

Additionally, this substance is responsible for modulating cell migration and proliferation, regulating inflammation control and, as a result, helps wound healing or repair. With advancing age and the decrease in the production of this substance, deficiencies are observed due to the inability to provide sufficient water to the tissues in the body. Therefore, it is recommended to use HA in hydrogel form with appropriate physical and mechanical properties for the treatment and improvement of such deficiencies.[23]

In the medical industry, HA has applications such as perurethral injection to treat urinary incontinence due to sphincter insufficiency, postoperative injection to prevent peritoneal adhesions, and injection to replace insufficient biological fluids (especially in the joints). HA's uses in the beauty industry include filling fine lines, wrinkles and skin imperfections and increasing volume.

Despite HA's unique properties and high compatibility with human body tissue, the raw (non-crosslinked) form of this material has prompted researchers to modify the structure of this material due to its less than favorable mechanical properties, low half-life, and high degradation rate. Some of the studies conducted for HA stability are described below.

HA physical modification was considered at first. Physical modification is done in two ways: increasing the entanglement of the polymer chains in solution and also forming the coacervation complex. Both of these methods lead to increased HA durability and improved properties. [24]

In a patent they received in 2013, Mario De Rosa and his colleagues investigated the properties of high molecular weight (H-HA) and low molecular weight (L-HA) complexes and their use in medicine, cosmetics and food.

In this claim, by using very high and low molecular mass and using a temperature cycle, they were able to form complexes with weak intermolecular forces, such as hydrogen bonds or hydrophobic interactions, resulting in very stable interactions between molecules.[25]

However, despite the advantages of physical modification, this method cannot form a high-strength hydrogel because the networks formed in this process will fail due to the application of stress. Other disadvantages of this method include the lack of resistance to enzymatic degradation of modified HA and its poor performance in aqueous environments.

Various crosslinking agents are used to chemically crosslink HA and create a three-dimensional structure in the polymer matrix. Substances used as crosslinking agents in recent years include 1,4-butanediol diglycidyl ether (BDDE), Methacrylamide, Hydrazide, Carbodiimide, Divinyl sulfone (DVS) and Poly(propylene glycol) diglycidyl ether.[26-27]

In 1986, Endre and colleagues studied HA cross-linking in the presence of the cross-linking agent DVS at 20°C. HA cross-linking with DVS is fast and highly efficient, but the high reaction rate has made it difficult to control the properties and homogeneous distribution of the cross-linking in the entire structure, and on the other hand, the high dissociation and also the toxicity of the bond connecting DVS with HA have made the commercialization of products in this category not well received. has happened. The Hylaform series from Genzyme, the Varioderm series from Adoderm, and the Teoxane series from Teosyal have used DVS as a binder to prepare HA gel. [28]

In other studies, Daniel Aeschlimann and Gabriel N. Njikang presented a method for modification of HA with different functional groups such as aldehyde and amine, allowing cross-linking of HA derivatives. [29] However, the difficulty and multi-stage nature of the reaction and the use of different toxic substances to reach the final product are among the disadvantages of research on industrialization in the field of medicine.

The Allergan Company's research team, Estelle Piron and her colleagues, investigated the reaction of HA with epoxy group in 2000. Since epoxy rings are under angular pressure, they have a high potential to react with hydroxyl groups. In this invention, mechanical properties of Diepoxies with different chain lengths according to various types were investigated.

These crosslinking agents include BDDE, 1,2-bis(2,3-epoxypropyl)-2,3-ethylene, and 1,4-Bis((2,3-epoxypropoxy)methyl)cyclohexane. The cross-linking agent is the best option according to the results obtained from BDDE.[30-31]

Therefore, researchers' interest in this cross-linking agent has increased day by day, so much so that most of the cross-linked HA-based products on the market today are cross-linked by BDDE.

For example, Restylane from Galderma [32], Juvederm from Allergan [33], Stylage from Vivacy [34] all used BDDE as a cross-linking agent to prepare HA gel. In these inventions, everyone tried to improve production conditions to obtain gels with desired properties. As a result, different products with different technologies are supplied to the world market.

The reasons for choosing BDDE as the main crosslinking agent to increase the physical and mechanical properties and stability of the produced hydrogels include its easy synthesis, less toxicity, and hydrolysis of epoxy groups into simple diols. Preparation of HA-based hydrogels with BDDE generally leads to the production of monophasic and biphasic products. The most important difference between these two technologies is the stickiness of the final product.[35]

In general, the preparation of cross-linked HA-based hydrogels involves mixing a cross-linking agent with HA to obtain cross-linked HA and purifying the final product. The cross-linking reaction is carried out in an alkaline environment, and the most common method for the purification step is the use of a dialysis bag with the aid of washing using water or buffer solution. [36]

Dialysis is a separation technique that facilitates the removal of small and undesirable compounds from macromolecules in solution by selective and passive diffusion through a semi-permeable membrane. In this method, a sample and a buffer solution are placed on opposite sides of the membrane. The purification method is such that molecules larger than the membrane pores remain on the sample side of the membrane, but small molecules and buffer salts pass freely through the membrane, reducing the concentration of these molecules in the sample [37-38]. Using this method is very expensive and time consuming. Therefore, there is a need to explore the use of other less expensive methods.

One strategy to eliminate the crosslinking agent is to extend the reaction time, presented by Ikada and colleagues and Li-Su Chen and colleagues from Scivision Biotech Inc. [39-40] However, this proposal requires increased reaction time and HA in alkaline conditions (pH). This claim has not been confirmed because >13) decomposes over a long period of time.

KARLSSON and his teammates at Galderma company suggested and implemented the use of the first type of alcohol (methanol, ethanol and isopropyl alcohol) and the precipitation method (solvent-anti-solvent) as an alternative solution in 2014. In this method, after cross-linking HA was formed, precipitation was carried out with the first type of alcohol and then purification was carried out in a combined solution consisting of ethanol and base for 45 hours to obtain optimal properties.[41]

However, the time-consuming and costly drawback of using a dialysis bag is also seen in this method. Moreover, the addition of alkali during the 45-hour (long time) purification step causes the pH to increase and, as a result, the uncontrolled destruction of the ether bonds of the HA polymer chains and finally the decrease in the stability of the final product.

It is, therefore, the object of the present invention to provide a method for preparing a cross-linked hyaluronic acid gel overcoming drawbacks of the prior art, especially to shorten the time of the manufacturing process, reduce the amount of crosslinking agent, such as BDDE, to prevent cross-network HA degradation, and to obtain a stable cross-Network HA powder for long-term storage and preservation that can be used for various applications.

SUMMARY OF THE INVENTION

The object is achieved by a method for preparing a cross-linked hyaluronic acid gel in accordance with claim 1. Further embodiments result from the subclaims and the following detailed description.

A first aspect of the invention provides a method for preparing a cross-linked hyaluronic acid gel comprising the steps:

- cross-linking hyaluronic acid with a cross-linking agent to obtain cross-linked hyaluronic acid;
- adding the cross-linked hyaluronic acid to an alcohol to obtain a first reaction mixture;
- adding the first reaction mixture to an alkaline alcoholic solution to obtain a second reaction mixture;
- separating at least one sediment from the first reaction mixture and/or at least one sediment from the second reaction mixture;
- treating the combined sediments with a buffer having a phosphate buffer to obtain the cross-linked hyaluronic acid gel

In a first implementation of the method according to the first aspect, the hyaluronic acid has a weight average molecular weight from 1,000,000 to 3,000,000 g/mol measured by gel permeation chromatography.

In a further implementation of the method according to the first aspect, the crosslinking agent comprises at least two epoxy-groups.

In a further implementation of the method according to the first aspect, the crosslinking agent is 1,4-butanediol diglycidyl ether.

In a further implementation of the method according to the first aspect, the amount of the crosslinking agent is such that the degree of transverse connections in the cross-linked hyaluronic acid is from 0.05 to 0.3.

In a further implementation of the method according to the first aspect, the crosslinking is performed under alkaline conditions.

In a further implementation of the method according to the first aspect, the alcohol is ethanol.

In a further implementation of the method according to the first aspect, the alkaline alcoholic solution is a solution of NaOH in ethanol.

In a further implementation of the method according to the first aspect, the method comprises a further step of drying the combined sediments before treating the combined sediments with the buffer.

In a further implementation of the method according to the first aspect, a weight ratio of hyaluronic acid to cross-linking agent in the cross-linking step is from 100:1 to 20:1.

In a further implementation of the method according to the first aspect, treating the combined sediments with a buffer having a phosphate buffer to obtain the cross-linked hyaluronic acid gel is performed with a concentration of cross-linked hyaluronic acid in the buffer from 16 to 28 mg/ml.

BRIEF DESCRIPTION OF THE DRAWINGS

To illustrate the technical features of embodiments of the present invention more clearly, the accompanying drawings provided for describing the embodiments are introduced briefly in the following. The accompanying drawings in the following description are merely some embodiments of the present invention, modifications on these embodiments are possible without departing from the scope of the present invention as defined in the claims.

FIG. 1 is a chromatogram of a blank solution,

FIG. 2 is a chromatogram of the system suitability solution,

FIG. 3 is a chromatogram of BDDE standard solution (0.01 mg/L),

FIG. 4 is a chromatogram of BDDE standard solution (0.02 mg/L),

FIG. 5 is a chromatogram of BDDE standard solution (0.2 mg/L),

FIG. 6 is a chromatogram of BDDE standard solution (1.0 mg/L),

FIG. 7 is a chromatogram of BDDE standard solution (2.0 mg/L),

FIG. 8 is a chromatogram of BDDE standard solution (4.0 mg/L),

FIG. 9 is a chromatogram obtained in C20 sample solution,

FIG. 10 is a chromatogram obtained in A10 sample solution,

FIG. 11 is a cchromatogram obtained in A20 sample solution,

FIG. 12 is a chromatogram obtained in sample solution 1,

FIG.13 is a calibration curve of areal density of the standard solutions,

FIG. 14 shows examination of the linear viscoelastic region (Storage modulus and loss modulus),

FIG. 15 shows the rheological behavior (complex module) for examples 1 and 2 (final product),

FIG. 16 is a rheological diagram of the gel prepared from C10 powder with a concentration of 24 mg/ml (Storage modulus and loss modulus),

FIG. 17 is a rheological diagram (complex module) of the gel prepared from C10 powder with a concentration of 24 mg/ml,

FIG. 18 is a rheological diagram of the gel prepared from C15 powder with a concentration of 24 mg/ml (Storage modulus and loss modulus),

FIG. 19 is a rheological diagram (complex module) of the gel prepared from C15 powder with a concentration of 24 mg/ml,

FIG. 20 is a rheological diagram of the gel prepared from C20 powder with a concentration of 24 mg/ml (Storage modulus and loss modulus),

FIG. 21 is a rheological diagram (complex module) of the gel prepared from C20 powder with a concentration of 24 mg/ml,

FIG. 22 is a rheological diagram of the gel prepared from C30 powder with a concentration of 24 mg/ml (Storage modulus and loss modulus),

FIG. 23 is a rheological diagram (complex module) of the gel prepared from C30 powder with a concentration of 24 mg/ml,

FIG. 24 is a rheological diagram of the gel prepared from C40 powder with a concentration of 24 mg/ml (Storage modulus and loss modulus),

FIG. 25 is a rheological diagram (complex module) of the gel prepared from C40 powder with a concentration of 24 mg/ml,

FIG. 26 is a rheological diagram of the gel prepared from D10 powder with a concentration of 24 mg/ml (Storage modulus and loss modulus),

FIG. 27 is a rheological diagram (complex module) of the gel prepared from D10 powder with a concentration of 24 mg/ml,

FIG. 28 is a rheological diagram of the gel prepared from D15 powder with a concentration of 24 mg/ml (Storage modulus and loss modulus),

FIG. 29 is a rheological diagram (complex module) of the gel prepared from D15 powder with a concentration of 24 mg/ml,

FIG. 30 is a rheological diagram of the gel prepared from D20 powder with a concentration of 24 mg/ml (Storage modulus and loss modulus),

FIG. 31 is a rheological diagram (complex module) of the gel prepared from D20 powder with a concentration of 24 mg/ml,

FIG. 32 is a rheological diagram of the gel prepared from D30 powder with a concentration of 24 mg/ml (Storage modulus and loss modulus),

FIG. 33 is a rheological diagram (complex module) of the gel prepared from D30 powder with a concentration of 24 mg/ml,

FIG. 34 is a rheological diagram of the gel prepared from D40 powder with a concentration of 24 mg/ml (Storage modulus and loss modulus),

FIG. 35 is a rheological diagram of the gel prepared from D40 powder with a concentration of 24 mg/ml (complex module),

FIG. 36 shows storage modulus and loss modulus versus time change for example 3, substep C and D at constant frequency (1Hz).

FIG. 37 shows yellowing of cross-linked hyaluronic acid (CL-HA) powder in example 2,

FIG. 38 is a complex module rheological diagram of the example 0 to 120 days,

FIG. 39 is an electron microscopic image showing the morphology of CL-HA powder at 0 hour;

FIG. 40 is an electron microscopic image showing the morphology of CL-HA powder at 20 hours.

In context with the figures, the number following the designation of the sample (A, B, C, D, E, F, and G), for example the number 10 in A10, refers to the immersion time in hours, for example 10, 20, and 30 hours etc. For example, A10 refers to a sample obtained in substep (A) of Example 2 with an

TECHNICAL EFFECT

Especially the following technical effects are achieved by the method for preparing a cross-linked hyaluronic acid gel in accordance with the present invention:

- Reduce network agent consumption.
- Eliminating the costly and time-consuming process of using a dialysis bag.
- Obtaining stable cross-linked HA powder.
- Improving the physical-mechanical properties of the final product.

DETAILED DESCRIPTION OF EMBODIMENTS

According to a first aspect, the present invention relates to a method for preparing a cross-linked hyaluronic acid gel. The method comprises a sequence of process steps which are carried out in the order as given in claim 1, wherein it is not excluded that the process steps may partially overlap.

The first step of the method for preparing a cross-linked hyaluronic acid gel is cross-linking hyaluronic acid with a cross-linking agent to obtain cross-linked hyaluronic acid. Crosslinking is the method by which one polymer chain is connected to another polymer chain or to another part of the same polymer chain, typically by a covalent bond.

Hyaluronic acid also called hyaluronan, is an anionic, nonsulfated glycosaminoglycan having the following formula

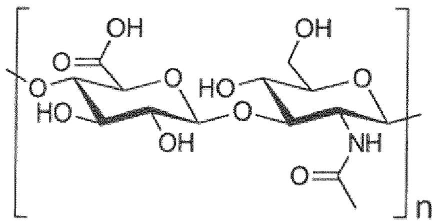

wherein "n" determines the number of repeating units and in this way the weight average molecular weight. The hyaluronic acid used in the inventive method may have a weight average molecular weight from 1,000,000 to 3,000,000 g/mol, preferably 1,500,000 to 2,500,000 g/mol, measured by gel permeation chromatography under the conditions as disclosed in Preis et al. Application Brief Materials Testing & Research, Agilent, True Answers 5994-5701EN published in 2023.

The term cross-linking agent as used herein may refer to a molecule which bridges two polymer molecules (or two different parts of the same polymer molecule) during cross-linking. The cross-linking agent as used in the method according to the present invention may be selected from the group consisting of a crosslinking agent comprises at least two epoxy-groups (bisepoxide), poly(ethyleneglycol) diglycidyl ether, poly(propylene glycol) diglycidyl ether, hydrazine, divinylsulfone (DVS), carbodiimide, methacrylamide, 1,4-butanediol diglycidyl ether (BDDE) and mixtures of two or more thereof, preferably may be 1,4-butanediol diglycidyl ether (BDDE).

The amount of the crosslinking agent in the cross-linking step may be such that the degree of transverse connections in the cross-linked hyaluronic acid is from 0.05 to 0.3, such as from 0.05 to 0.13, preferably from 0.05 to 0.07. In this regard, the degree of transverse connections "R" in the cross-linked hyaluronic acid is defined according to the following formula

$$R = \frac{(number\ of\ moles\ of\ cross-linking\ agent\ introduced\ into\ the\ cross-linked\ hyaluronic\ \mathrm{acid})}{(number\ of\ moles\ of\ disaccharide\ unit\ in\ the\ cross-linked\ hyaluronic\ acid)}$$

A weight ratio of hyaluronic acid to cross-linking agent in the cross-linking step is from 100:1 to 20:1. The crosslinking may be performed under alkaline conditions. The crosslinking may be performed by mixing contacting HA and crosslinking agent, such as BDDE, in an alkaline aqueous solution. The alkaline aqueous solution may be a 0.1 N to 1N, such as an 0.25 N NaOH solution. The crosslinking may be performed at a temperature from room temperature (such as 23°C) to 100°C, preferably at a temperature from 30 to 50°C, such as about 42°C. The crosslinking may be performed for 10 min to 24, preferably for 1 to 5 h, such as about 3 h.

The second step of the method for preparing a cross-linked hyaluronic acid gel is adding the cross-linked hyaluronic acid obtained in the first step to an alcohol to obtain a first reaction mixture. The cross-linked hyaluronic acid may be added to the alcohol under stirring, such as mechanical stirring. The alcohol may be a primary alcohol, preferably selected from the group consisting of methanol, ethanol, and propanol, most preferred ethanol. The cross-linked hyaluronic acid may be added to the alcohol in an amount of 0.01 g to 0.5 g, such as about 0.02 g, with respect to 1

ml of alcohol.

The third step of the method for preparing a cross-linked hyaluronic acid gel is adding the first reaction mixture obtained in the second step to an alkaline alcoholic solution to obtain a second reaction mixture. The first reaction mixture may be added to the alcohol under stirring, such as mechanical stirring. The alcohol in the alkaline alcoholic solution may be a primary alcohol, preferably selected from the group consisting of methanol, ethanol, and propanol, most preferred ethanol. The alkaline alcoholic solution is a solution of NaOH in ethanol, preferably a 0.1 N to 1 N, such as about 0.25 N, NaOH/EtOH solution. The first reaction mixture is added to the alkaline alcoholic solution in an amount that the amount of cross-linked hyaluronic acid to the alkaline alcoholic solution in an amount of 0.01 g to 0.5 g, such as about 0.02 g, with respect to 1 ml of alcohol.

The fourth of the method for preparing a cross-linked hyaluronic acid gel is separating at least one sediment from the first reaction mixture and/or at least one sediment from the second reaction mixture. The separating may be precipitating the at least one sediment and/or at least one sediment, that is, letting the CL-HA precipitate. The solution above the sediment may be discarded slowly and then the sediment may be squeezed and the remainder may be placed to dry out.

The fifth step of the method for preparing a cross-linked hyaluronic acid gel is treating the combined sediments with a buffer having a phosphate buffer to obtain the cross-linked hyaluronic acid gel. The buffer having a phosphate buffer may be a phosphate buffer, preferably a phosphate buffer pH 6.0 to 8.0, that is a phosphate buffer suitable to adjust the pH of the treatment mixture to a pH in a range from 6.0 to 8.0.

Treating the combined sediments with a buffer having a phosphate buffer to obtain the cross-linked hyaluronic acid gel may be performed with a concentration of cross-linked hyaluronic acid in the buffer from 16 to 28 mg/ml.

When contacting the combined sediments (comprising the cross-linked hyaluronic acid) with the buffer, the cross-linked hyaluronic acid swells in the buffer and finally the cross-linked hyaluronic acid gel is formed.

The method may comprise a further step between the fourth step and the fifth step of drying the combined sediments before treating the combined sediments with the buffer. The combined sediments may be dried in a vacuum-oven at room temperature, for example, for a time span of about 10 to 40 hours. The combined sediments may be dried using a freeze-dryer for a time period from 1 to 48 hours, such as about 24 hours, at a temperature from 2°C to 8°C.

The method may comprise a further step of washing the final product under acidic conditions, such as washing with acidic ethanol.

In addition, the following detailed explanations and features may material for realizing the invention.

As mentioned, BDDE is the most commonly used crosslinking agent for HA chemical modification. The superior stability of the ether bond (compared to the ester or amide bond) is one of the reasons why BDDE cross-linked HA fillers can last 1 year or longer.

Despite all the advantages and widespread use of this substance, the high incorporation of this substance in the formulation used in HA-based products is the main concern of all researchers working in this field. The reason why BDDE is widely used in the production of HA gels is to improve the physical-mechanical properties of the final gel.

Allergan Company is one of the leaders in improving the networking process and reducing the consumption of networking intermediaries. Allergan introduced the first generation of their cross-linked HA gel product called Hylacross, based on HA network technology prepared from high molecular weight HA and BDDE in an alkaline environment and using a dialysis bag for purification.[42] In the next attempt to reduce BDDE consumption, it introduced a new technology called Vycross. By combining two high and low molecular weight HA masses, it managed to achieve the desired properties with less consumption of BDDE cross-linking agent and registered its claim as a success in reducing the amount of BDDE. In other studies, it is possible to mention the use of methods such as IPN to improve rheological properties and stable removal by using two cross-linked HA interpenetrations and making the cross-linking reaction two-stage. This method improved the rheological properties but is far from the goals we had in this claim. The use of high amounts of BDDE to improve the physical and mechanical properties of HA increases the possibility of an allergic reaction in the body after injection, as well as leading to high purification costs such as the use of a dialysis bag for several days.[43]

In the Hoshyar/High/Hyaluronic acid (HOLTECH) technology mentioned in this claim, these disadvantages have been eliminated and new advantages have been obtained that increase the ability to progress in this field. In HOL technology, the time-consuming step, the cost of the dialysis bag and the consumption of BDDE were reduced, as well as the reaction efficiency was increased and it was also possible to prepare stable HA cross-linked mixture for long-term storage that could be used at any time depending on the desired application of the cross-linked HA. Compared to the method proposed in the invention, it has been observed as a result of the research that HOLTECH technology has significant advantages such as reducing cost, reducing cross-linking agent consumption, reducing polymer degradation, storage capacity and stabilization of cross-linked powder as raw material in different applications.

In general, the process of obtaining a stable mesh powder is to use the solvent-anti-solvent process with the help of a first type of alcohol such as methanol and ethanol, and replace it with white fibers as the intermediate material. HA dissolves in its appropriate environment and after reacting with the cross-linking agent, the product is obtained as a solution containing cross-linked HA and by-products. In the first type of alcohol, cross-linked HA is insoluble, but other by-products are soluble, and by this trick HA powder can be easily separated from by-products.

In the case of the reaction of HA with BDDE, these factors can be strengthened by increasing the reaction temperature and creating the appropriate environment and reaction time, but these parameters have the opposite effect, causing the physical and mechanical properties to deteriorate and decrease.

When HA begins to react with the cross-linking agent, the chains bind to each other, causing the molecular mass to increase. The increase in molecular mass causes a decrease in chain mobility and steric hindrance, and the reaction efficiency decreases.

What is important is that when the HA network precipitates in the first type of alcohol, several things happen:

- By bringing the chains together the reaction is directed towards completion, in fact the reaction continues in the solid phase.
- Due to the hydrophobic structure formed by type primary alcohol, hydrophilic regions tend towards each other and this increases the possibility of reaction.

EXPERIMENTAL PART

Used materials

HA (1.5-2.5 million dalton) (Kikkoman Biochemifa Company), BDDE (Sigma Aldrich Company), Epicholorohydrin (Sigma Aldrich Company), Sodium diethyldithiocarbamate trihydrate (Sigma Aldrich Company), Potassium dihydrogen Phosphate (Merck company), Phosphoric Acid (85% Merck company), Ethanol (99.9% Merck company), Acetonitrile (99.9% Neutron Company), Sodium Hydroxide (Merck company), Carbazole (99.9% Sigma Aldrich company).

Various devices, tools and methods were used to analyze the properties of the prepared cross-linked hyaluronic acid (CL-HA) gels. Rheometer devices were used to investigate the viscoelastic properties of the gels. The samples were set and controlled in the frequency range from 0.01 to 100 Hz at 2% strain (strain in the linear viscoelastic region) using an Anton Paar MCR 102 model rheometer equipped with a CP25/2 spindle at 25°C. A 1 ml BD Hylok syringe with a 27G TSK brand PRC-13mm needle head was used.

A Lyomax 4S model cooler (freezer) was used for drying (lyophilization) of the powder.

HACH DR 6000 ultraviolet spectrometer was used to measure the cross-linked HA concentration and gel content in the final product.

HPLC technique was used to measure the remaining BDDE from the purification step. This analysis was performed with a Waters 2695 HPLC instrument with a PDA996 detector and an Agilent column with a length of 150 mm, a diameter of 4.6 mm and a particle size of 5 micrometers.

A scanning electron microscope model S360 manufactured by the Cambridge company was used to investigate the structural morphology of CL-HA powder.

According to the purposes stated in this invention, the following 5 experiments were designed and their results were examined in comparison with the traditional method of producing CL-HA gels.

When the studies are examined, the parameter called the degree of transverse connections (R) is expressed according to the following equation:

$$R = \frac{(number\ of\ moles\ of\ cross-linking\ agent\ introduced\ into\ the\ cross-linked\ hyaluronic\ \mathrm{acid})}{(number\ of\ moles\ of\ disaccharide\ unit\ in\ the\ cross-linked\ hyaluronic\ acid)}$$

Example 1 (comparative) using dialysis for purification

10 grams of HA was dissolved in 60 ml (0.25N) NaOH solution. After HA was dissolved, 0.39 grams of BDDE (R = 0.13) was added. The reaction was carried out at 42°C for 3 hours. The reaction mixture was then dialyzed against phosphate buffer for 72 h so that neutralization and purification could be performed simultaneously. The final gel was prepared at a concentration of 24 mg/ml.

Example 2

10 grams of HA was dissolved in 60 ml (0.25 N) NaOH solution. After HA was dissolved, 0.39 grams of BDDE (R - 0.13) was added. The reaction was carried out at 42°C for 3 hours.

Substep (A): The reaction mixture was added to the EtOH tank under mechanical stirring.

Substep (B): The reaction mixture was added to the NaOH (0.25):EtOH tank under a mechanical stirrer.

The sediment from substeps (A) and (B) was separated and dried after 10, 20, 30, 40 hours (immersion time). Then, the final gel was prepared at a concentration of 24 mg/ml after the desired time using phosphate buffer.

Example 3 (Optimizing the amount (value) of R)

10 grams of HA was dissolved in 60 ml (0.25N) NaOH solution. After HA was dissolved, 0.18 grams of BDDE (R = 0.06) was added. The reaction was carried out at 42°C for 3 hours.

Substep (C): The reaction mixture was added to the EtOH tank under mechanical stirring.

Substep (D): The reaction mixture was added to the NaOH(0.25):EtOH tank under mechanical stirring.

The sediment from substeps (C) and (D) was separated and dried after 10, 15, 20, 30, 40 hours (immersion time). Then, the final gel was prepared at a concentration of 24 mg/ml after the desired time using phosphate buffer.

Example 4 (Investigation of long-term stability of sediment)

In this example, the physical-mechanical properties of the C20 precipitate (sediment of substep C obtained with 20 hours immersion time) were investigated in order to check long-term stability.

Substep (E): It was dried using a freezer and stored at 2-8 °C.

Substep (F): It was washed with HCl-EtOH solution and dried under vacuum in a normal oven and stored at 2-8 °C.

Substep (G): It was washed with HCl:EtOH solution, dried using a freezer, and stored at 2-8 °C.

To check the properties of all prepared sediments (sediments) after the desired periods (0-120 days), the final gel was prepared at a concentration of 24 mg/ml using phosphate buffer.

Example 5 (Preparation of gel at different concentrations)

The cross-linked powder of example H was prepared in phosphate buffer with concentrations of 16, 20, 24 and 28 mg/ml.

As mentioned before, the most comprehensive aim of this invention is to obtain a stable CL-HA powder at the lowest cost and in the highest quality and to prepare gels that can be commercialized and used in desired medical applications. In fact, with this work, a substance that can be used as raw material for various industries is prepared.

To achieve the stated objectives of the invention, examples (experiments) were designed to finally arrive at the best method and formulation to commercialize products in this category. The sequence of experiments is first to evaluate the effectiveness of the purification method, then the effectiveness of the reaction and achieving the desired properties, and finally the stability of the CL-HA powder and the preparation of the gel with different properties.

The most important challenge in making products used in medical applications and placed in the body is the absence of toxicity and impurities (presence of mixing). The most important impurity present in the HA cross-linking process is the unreacted BDDE residue. The first aim of this invention is to use a new purification system instead of the old time-consuming and expensive dialysis bag method. The basis of the method used is based on solubility difference. In this way, BDDE is soluble in ethanol, while CL-HA is insoluble in ethanol, and due to the difference in this behavior, they can be completely decomposed at high speed and in the shortest time. After the reaction of HA with BDDE, the resulting product is added to the ethanol tank, forming CL-HA powder in ethanol, but BDDE is soluble in ethanol and can be easily separated from CL-HA. To quantitatively check the effectiveness of this method, HPLC was used to determine the amount of BDDE based on the obtained data. The amount of BDDE remaining in the gels prepared in samples A10, A20 and C20 is much lower than in the gel in the first sample. Therefore, this method is much better than the traditional dialysis bag method in terms of efficiency, time, cost and commercialization. HPLC plots are shown in Figures 1 to 13. So far, the dialysis bag method has been removed from the process.

Another important issue encountered in gels is that the gels have mechanical properties and stability for the intended application. Rheology tests and concepts of rheology science were used to investigate the behavior against mechanical stress. Since the behavior of gels is derived from the result of elastic and viscous behavior, this category of gels is known as viscoelastic fluids. In order to accurately compare the behavior of gels, the linear viscoelastic region must first be determined. According to the test performed at different pressures, it was found that 2% pressure was in the linear viscoelastic region and all rheological property tests were performed at this pressure (Figure 14).

In network structured hydrogels, the elastic part of the gel should be higher than the viscous part. Depending on the configuration and conformation, the amount of viscoelasticity returns as rotation-vibration and translational movements of the polymer, causing the amount of pressure applied to the gel to be lost or deposited. Storage modulus (G') is a symbol of gel elasticity. For each sample, G' gradually increases with increasing angular frequency. At lower frequencies the sample has sufficient time to relax and the polymer chains are capable of deforming against the applied force, but at higher frequencies the polymer chains do not have sufficient time to relax and G' increases. The loss modulus (G"), which

represents viscous behavior, arises from internal friction between the components of a flowing liquid as a result of intermolecular and interparticle interactions. This friction is always associated with the generation of frictional heat in the sample and the resulting conversion of frictional energy into thermal energy. This part of the energy is absorbed by the sample and consumed by internal friction processes. The value of G also increases with increasing frequency. The complex modulus (G*) obtained as a result of the two quantities G' and G" using Equation 1 shows the resistance of the fluid to the deformation of the sample[44].

$$G^{*}=\sqrt{(G')^2+(G")^2} \quad equ1$$

To make a better comparison, the modulus amount was announced at 1 Hz frequency, 25°C and 2% strain. The origin of the mechanical strength of a hydrogel is generally based on the formation of transverse connections and nodes in the structure of the hydrogel, such that the number of segments increases and their length decreases as the network increases. This causes the amount of permanent deformation to decrease when stress is applied to the polymer structure, and it returns to its former order after the stress is removed. Other tests to prove reticulation include measuring gel content and swelling of the gel. As reticulation increases, the percentage of gel content increases and the amount of swelling decreases. As cross-linking increases and more HA chains are incorporated, the number of free chains decreases. On the other hand, as the network density increases, it becomes more difficult for water molecules to penetrate the polymer tissue and the shortness of the segments prevents swelling. Increasing the G*, increasing the gel content, reducing the swelling and reducing the sol, all indicate greater stability of the gels within the body.

According to Equation 1, G' is inversely related to the average molecular weight between the two cross-links (Mc) forming the segment. An increase in Mc is equivalent to a decrease in the density of transverse connections and an increase in swelling. It is quite obvious that swelling and gel strength have an inverse relationship; The higher the swelling, the lower the gel strength. A decrease in Me means higher cross-link density and leads to less swelling, resulting in increased strength of the gel.[45]

$$G' = \frac{\rho RT}{M_C} \qquad equ\ 2$$

Therefore, if the amount of covalant bond and ties increases, the weight of the segment will decrease, the number of segments will increase and G' will be high. Therefore, by examining various rheological properties of the gel, different parameters can be interpreted and analyzed as follows:

$$CLD \ \acute{\alpha}\ G' \ \acute{\alpha}\ \frac{1}{Mc}\ \acute{\alpha}\ N.S\ \acute{\alpha}\ \frac{1}{SWD}$$

CLD = Cross-Linked Density

G' = Storage Modulus

Mc = Molecular Weight between Crosslinking Point

N. S= Number of Segment

SWD= Swelling Degree

In the HA cross-linking reaction, HA first dissolves in alkaline media. This solution causes the nodes in the chain to unravel. Crosslinking agent is then added to the solution to initiate the crosslinking reaction. However, the destruction of HA chains along with the cross-linking reaction occurs due to the alkalinity of the environment and competes with the cross-linking reaction. In all examples the initial stages of the reactions are similar, but in example 1 a dialysis bag is used for neutralization and purification after the reaction, which stops the cross-linking and degradation reaction due to neutralization of the medium.

In the case of example 2, purification is done in ethanol because unreacted BDDE is dissolved in ethanol and separated from the CL-HA tissue. On the other hand, precipitating CL-HA in ethanol medium and leaving CL-HA in this state causes the additional cross-linking reaction to continue in the solid phase. Another thing that happens as a result of sedimentation is the aggregation of polymer strands, the formation of knots and the intertwining of the polymer structure. In example 2, the reaction continues until the polymer arrived to a strong network structure. The density of transverse

connections increases strongly, Mc and water absorption decreases. This happens faster when the ethanol is alkaline because the alkaline environment causes the reaction to proceed. Therefore, B2 gel reaches the biphasic and lumpy state earlier than A2 and loses its gel properties. However, at the end of example 2, the reaction progresses to the point of losing the gel state. Comparing the data of example 1 and 2 shown in Figure 15 and Table 1, it is seen that the gel prepared in example 2 it is quite obvious that it has a higher G* (mechanical properties) and gel content and a lower Mc and swelling compared to sample 1, confirming that the network process is not completed in the first step.

**Table 1.** Rheological results, swelling degree, gel content and molecular weight of the fragments for different states of Samples 1 and 2.

| Sample | G' (Pa) | G" (Pa) | (Tan δ) | \|η*\| (Pa.s) | G* (Pa) | SWD | Gelc (%) | Mc (kg/mol) |
|---|---|---|---|---|---|---|---|---|
| Ex1 | 543.28 | 80.86 | 0.148 | 87.41 | 551 | 154 | 81 | 4.92 |
| A10 | 902 | 171 | 0.19 | 146 | 915 | 76 | 86 | 2.96 |
| A15 | 1030 | 165 | 0.16 | 166 | 1043 | 72 | 93 | 2.59 |
| B10 | 2675 | 460 | 0.17 | 432 | 2714 | 55 | 91 | 1.01 |

However, a more complete reaction and higher properties were achieved with the technology investigated in this invention. Traditionally, the density of cross-links is easily increased with large amounts of cross-linking agent. A wide variety of studies have been conducted on the concentration of the crosslinking agent and the properties of hydrogels and other types of polymers. However, the purpose of this invention is to reduce the consumption of the network agent and increase efficiency. Therefore, by using technology of this invention to reduce the cross-linking agent in the first stage and increase the efficiency of cross-linking, these changes can be better controlled and more suitable properties can be obtained.

Therefore, in Example 3, the amount of crosslinking agent was reduced to less than half and the progress of the reaction was controlled in the presence and absence of alkali to determine the optimal time and conditions. In example 2, the alkaline environment observed better the behavior seen in the presence or absence of ethanol. In example 3, substep C, sedimentation was done in ethanol solution and in substep D, sedimentation was done in alkaline ethanol, samples were taken after immersion time between 10 and 40 hours and a gel was prepared from them at a concentration of 24 mg/ml. The results are shown in Figures 16 to 36 and Table 2.

**Table 2.** Rheological results of samples for different states of the sample, degree of swelling, gel content and molecular weight.

| Sample | G' (Pa) | G'' (Pa) | (Tan δ) | \|η*\| (Pa.s) | G* (Pa) | SWD | Gelc (%) | Mc (kg/mol) |
|---|---|---|---|---|---|---|---|---|
| C10 | 87 | 41 | 0.477 | 15.3 | 89.65 | 120 | 95 | 30.75 |
| C15 | 363 | 79 | 0.22 | 59 | 376 | 129 | 92 | 7.37 |
| C20 | 537 | 80 | 0.15 | 86 | 543 | 143 | 98 | 4.98 |
| C30 | 592 | 125 | 0.21 | 97 | 598 | 170 | 94 | 4.51 |
| C40 | 615 | 171 | 0.27 | 101 | 628 | 176 | 95 | 4.35 |
| D10 | 109 | 31 | 0.29 | 18 | 114 | 58 | 88 | 24.54 |
| D15 | 454 | 161 | 0.35 | 76 | 469 | 54 | 92 | 5.89 |
| D20 | 391 | 135 | 0.34 | 66 | 399 | 72 | 95 | 6.84 |
| D30 | 360 | 226 | 0.62 | 67 | 368 | 76 | 95 | 7.43 |
| D40 | 363 | 189 | 0.52 | 65 | 364 | 88 | 90 | 7.37 |

As it is observed that the reaction progresses in D compared to C due to the effect of the catalyst environment in the development of the reaction the rheological results of gels prepared from CL-HA powder soaked in ethanol for 10 hours are compared.

If the ethanol environment is alkaline and rheology parameters are high, water absorption is lower and Mc is lower, indicating that the progress of the reaction increases with the alkalinity of the environment. The same process continues for 15 hours, but as more time passes, a decrease in rheological properties may be observed in the sample in alkaline ethanol. Additionally, the amount of Mc and its swelling also increase, which is the reason for the cross-linking

reaction in the first 15 hours. Then, degradation of CL-HA in alkaline environment dominates, which is not seen in C due to the use of pure ethanol. In D, the amount of "tan delta" increases after 20 hours and G' decreases, and the difference between G' and G" also decreases, indicating the dominance of the degradation reaction. At the same time, after 20 hours in the gel prepared from mode C, G' and G' The amounts increased with a slight slope, indicating that the reaction was completed and the elasticity of the gel was maintained. After 25 hours, yellowing of the color of the precipitated powder can be seen in powder D, which is caused by the degradation of CL-HA. (Figure 37).

There is no change in the properties of the final gel up to 45 hours. Therefore, using ethanol instead of base ethanol is economically better, the reaction rate is more controlled, and the degradation of CL-HA occurs less. Comparison with the gels in A and B demonstrates the efficiency of the method presented in this technology. Conventional methods to increase the properties of gels rely on adding more BDDE to the reaction medium. However, in this invention, the desired and higher properties were obtained by reducing BDDE consumption and increasing the reaction efficiency. On the other hand, by purifying the alkaline environment from ethanol, the reaction steps are made easier and more economical, and the destruction of CL-HA in a way that does not cause degradation is prevented by achieving higher properties. Until this stage, the method of making C20 powder had been chosen as the most suitable method for making all kinds of gels.

Another object of this invention is to stabilize the CL-HA powder as a commercial product from which the consumer can prepare a gel of any concentration. In this context, example 4 was designed and the long-term (120 days) stability of the powder for storage was checked. The C20 powder prepared in parts E was dried by freeze-drying and kept at 2-8 °C'.After 10 days, the color of the powder turned completely yellow, which is an indication that the powder has deteriorated. The results obtained in Examples 2 and 3 confirmed that the environment caused degradation of CL-HA.

Since the reaction of BDDE with HA is carried out in an alkaline environment and CL-HA becomes alkaline when added to ethanol, the polymer chains are ionic. It was stated that the characteristics of the environment caused the CL-HA powder structure to be destroyed within a 10-day period. Therefore, in parts F and G, the CL-HA powder was washed with acidic ethanol to neutralize CL-HA. Then, the effect of drying in a normal room and with a freezer on the stability of CL-HA powder in storage was investigated.

The results of the tests performed in the time intervals 0 to 120 days are shown in Figure 38 and Table 3 Comparing F and G substep, the freeze-drying is more stable in the long term as the drying power is higher and less moisture remains in the powder.

**Table** 3. Example Rheological results, swelling degree, gel content and molecular weight of the relevant samples in different conditions and several days(o to 120 days).

| Sample | G' (Pa) | G" (Pa) | G* (Pa) | SWD | Gelc (%) | Mc (kg/mol) |
|---|---|---|---|---|---|---|
| E0 | 541 | 124 | 555 | 150 | 92 | 4.94 |
| F0 | 566 | 146 | 584 | 152 | 95 | 4.72 |
| F10 | 540 | 164 | 565 | 147 | 94 | 4.95 |
| F30 | 438 | 86 | 447 | 136 | 93 | 6.10 |
| F60 | 391 | 135 | 414 | 200 | 95 | 6.84 |
| F120 | 356 | 41 | 358 | 203 | 96 | 7.43 |
| G0 | 523 | 78 | 528 | 151 | 98 | 5.11 |
| G10 | 515 | 105 | 525 | 152 | 95 | 5.19 |
| G30 | 584 | 165 | 607 | 151 | 95 | 4.58 |
| G60 | 592 | 129 | 608 | 150 | 94 | 4.51 |
| G120 | 566 | 95 | 573 | 148 | 98 | 4.72 |

However, in case of drying in a normal oven, the moisture content is higher and more property loss is observed as it increases the possibility of destruction, but this rate is still not serious and the percentage of property loss is almost less than 15%. The results of this example show that with this technology, stable CL-HA powder can be prepared easily and at the lowest cost, it can be used industrially and commercially, this substance can be used in dermatology, cosmetology, ophthalmology, surgery and healing, urology, otolaryngology, rheumatology and osteomurritis treatment, etc.

It proved that it can be used as raw material in the production of various types of gels for medical applications such as gels, etc., and that it can be used by dissolving it in different amounts of powder buffer solutions depending on the desired properties.

It was designed to investigate the effect of HA concentration on the rheological properties of Example 5. From it 16

to 28 concentrations should be prepared and compared. As one can see in Figure 39, rheological properties also increase with increasing concentration. Therefore, depending on the place of use of the final gel in the body, a gel at a specific concentration can be easily prepared by adding CL-HA powder to the buffer.

Initially, precipitate preparation under stirring will probably be more efficient. While adding CL-HA solution to ethanol, mixing was done with a mechanical mixer. With this work, purification and precipitation are carried out evenly and quickly. To check the morphology of the CL-HA powder, two photographs of the C powder were taken under the microscope (SEM) while immersed in ethanol at 0 and 20 hours. As seen in Figures 39 and 40, both are in the form of fibers with a diameter of nanometers, and when the two images are compared, it is clearly seen that the number of holes decreases and the density increases when the time reaches from 0 to 20 hours. According to studies, there is a direct relationship between the degree of reticulation and the size of the holes formed on the CL-HA structure. 28-34 and these images prove that improved rheology properties over time.

Nanoscale fiber morphology has advantages such as increased surface area, increased buffer absorption rate that finally which leads to better solubility.

In order to further check that the gels prepared with the technology presented in this invention do not have any problems and complications for internal use, all samples in Example 5 were subjected to biological evaluation by Invitro and Invivo tests according to Table 4.

**Table 4:** Biological tests of In Vitro and In Vivo studies taken from samples

| Test name | Standard | Animal/cell line | Duration |
|---|---|---|---|
| MTT | ISO 10993-5 | L929 Fibroblast Cell | 24 hours |
| AMES | ISO 10993-3 | S. Typhimurium from the bacterial strains | 48 hours |
| 476 | OECD 476 | CHO-K1/An1 | 24 hours |
| Irritation | ISO 10993-23 | White New Zealand rabbit | 72 hours |
| Implantation early time | ISO 10993-6 | Wistar Albino Rat | 2 weeks |
| Implantation mid time | ISO 10993-6 | Wistar Albino Rat | 12 weeks |
| Implantation late time | ISO 10993-6 | Wistar Albino Rat | 24 weeks |
| Pyrogenicity | European Pharmacopoeia 2.6.8 | White New Zealand rabbit | 24 hours |
| Sensitization | ISO 10993-10 | Guinea Pig | 20 days |
| Acute toxicity | ISO 10993-11 | Mice CD1 | 72 hours |
| Sub-chronic toxicity | ISO 10993-11 | Mice CD1 | 90 days |

Results from analysis of cytotoxicity tests showed normal cell behavior and no changes in cell morphology. Therefore, no cellular or genetic toxicity occurred in these gels.

In animal tests conducted for up to 90 days, it was determined that acute and subacute phase toxicity did not occur. The animal's behavior and weight gain were considered normal. Hematogram and histopathology results were also normal. In animal studies, no tissue changes related to sensitivity or irritation at the injection site were observed during the study. The animal's behavior in terms of weighing and feeding was also normal. In subcutaneous implantation studies, no changes in the animal's tissue and behavior were observed at 2 weeks, 3 months and 6 months evaluation periods. According to the European Pharmacopoeia; The antipyretic test on this product was conducted on a New Zealand white rabbit, and changes in the animal's body temperature were evaluated within the normal range.

Application of the invention to industry

In this invention, HOLTECH technology is presented for the first time, which could revolutionize the industry of networked hyaluronic acid-based products. With this technology, the traditional purification method of the dialysis bag is eliminated, reducing cost and time while also achieving a perfect purification process. With this technology, the efficiency of the reaction has increased, which has led to a decrease in the consumption of cross-linking agent, at the same time the rheological properties have increased with a minimum amount of cross-linking agent, and most importantly, it is a stable product, which has the advantages of Nano and can be used as raw material in the production of various commercial gels. nano powder can be produced. With this technology, a product that is a raw material for various industries and requires gels with rheological properties is made for the first time. Additionally, drug-containing gels, subcutaneous injection gels, orthopedic gels, anti-adhesion gels, dressing gels, etc. Any gel is easy to prepare.

In this invention, a technology is presented that has an intelligently efficient purification process and enables the preparation of a nanopowder based on networked hyaluronic acid, which is stable and can be stored and used commercially (HOLTECH). Based on the designed samples and tests, this technology's purification method was proven to be more efficient than the dialysis bag method. By increasing the efficiency of the reaction, the consumption of cross-linking agent can be reduced and the properties can also be increased. It can be said that both purification and increasing the reaction yield are done in a single step. The alkaline environment increases the speed of completion of the reaction but also causes HA degradation. By adjusting alkaline conditions and drying the product, a stable powder can be easily prepared, which can be used to prepare a gel with different properties at different concentrations. They have no biological problems and are extremely compatible with the body. Due to its proven advantages, this technology can be used in systems based on natural polymers such as polysaccharides for cross-linking. It can be used as raw materials in various industries and formed in mid-level factories whose products are mesh powders (powder) with high physical and mechanical properties. This technology (HOLTECH) consists of a series of processes that ultimately lead to the production of the product.

The foregoing descriptions are only implementation manners of the present invention, the scope of the present invention is not limited to this. Any variations or replacements can be easily made through person skilled in the art. Therefore, the protection scope of the present invention should be subject to the protection scope of the attached claims.

## Cited literature

[1]. Sharma, S., & Tiwari, S.. A review on bio macromolecular hydrogel classification and its applications. International journal of biological macromolecules, (2020) 162, 737-747.

**[2].** Klein, J., Goldberg, R., luster, N., & Sorkin, R. (2019). U.S. Patent No. 10,314,946. Washington, DC: U.S. Patent and Trademark Office.

[3]. Sánchez-Cid, P., Jimenez-Rosado, M., Romero, A., & Pérez-Puyana, V. Novel trends in hydrogel development for biomedical applications: A review. Polymers, (2022), 14(15), 3023.

**[4].** Cascone, S., & Lamberti, G. Hydrogel-based commercial products for biomedical applications: A review. International journal of pharmaceutics, (2020), 573, 118803.

**[5].** Bashir, S., Hina, M., Iqbal, J., Rajpar, A. H., Mujtaba, M. A., Alghamdi, N. A., ... & Ramesh, S. Fundamental concepts of hydrogels: Synthesis, properties, and their applications. Polymers, (2020), 12(11), 2702.

**[6].** Govil, A., Jones, M. L., & Lubock, P. (2016). U.S. Patent No. 9,327,061. Washington, DC: U.S. Patent and Trademark Office.

[7]. Müller, A., Herbrechtsmeier, P., Knuth, M., & Nikolaus, K. (2014). U.S. Patent No. 8,647,271. Washington, DC: U.S. Patent and Trademark Office.

**[8].** Yang, J., Chen, Y., Zhao, L., Zhang, J., & Luo, H. Constructions and properties of physically cross-linked hydrogels based on natural polymers. Polymer Reviews, (2023), 63(3), 574-612.

**[9].** Pita-López, M. L., Fletes-Vargas, G., Espinosa-Andrews, H., & Rodriguez-Rodriguez, R. Physically cross-linked chitosan-based hydrogels for tissue engineering applications: A state-of-the-art review. European Polymer Journal, (2021), 145, 110176.

**[10].** Patterson, R., Kandelbauer, A., Müller, U., & Lammer, H. (2022). Cross-linked thermoplastics. In Handbook of thermoset plastics (pp. 719-756). William Andrew Publishing.

**[11].** Alavarse, A. C., Frachini, E. C. G., da Silva, R. L. C. G., Lima, V. H., Shavandi, A., & Petri, D. F. S. Crosslinkers for polysaccharides and proteins: Synthesis conditions, mechanisms, and crosslinking efficiency, a review. International journal of biological macromolecules, (2022), 202, 558-596.

**[12].** Muir, V. G., & Burdick, J. A. Chemically modified biopolymers for the formation of biomedical hydrogels. Chemical reviews, (2020), 121(18), 10908-10949.

**[13].** Peppas, N. A., & Mikos, A. G. Preparation methods and structure of hydrogels. In Hydrogels in medicine and pharmacy(2019), (pp. 1-26). CRC press.

**[14].** Pita-López, M. L., Fletes-Vargas, G., Espinosa-Andrews, H., & Rodriguez-Rodriguez, R. Physically cross-linked chitosan-based hydrogels for tissue engineering applications: A state-of-the-art review. European Polymer Journal, (2021), 145, 110176.

[15]. George, A., Sanjay, M. R., Srisuk, R., Parameswaranpillai, J., & Siengchin, S. A comprehensive review on chemical properties and applications of biopolymers and their composites. International journal of biological macromolecules, (2020), 154, 329-338.

**[16].** Moradali, M. F., & Rehm, B. H. Bacterial biopolymers: from pathogenesis to advanced materials. Nature Reviews Microbiology, (2020), 18(4), 195-210.

[17]. Muir, V. G., & Burdick, J. A. Chemically modified biopolymers for the formation of biomedical hydrogels. Chemical reviews, (2020), 121(18), 10908-10949.

[18]. Das, A., Ringu, T., Ghosh, S., & Pramanik, N. A comprehensive review on recent advances in preparation, physicochemical characterization, and bioengineering applications of biopolymers. Polymer Bulletin, (2023), 80(7), 7247-7312.

**[19].** Narins, R. S., & Bowman, P. H. Injectable skin fillers. Clinics in plastic surgery, (2005), 32(2), 151-162.

[20]. Calabro, A., Oken, M. M., Hascall, V. C., & Masellis, A. M. Characterization of hyaluronan synthase expression and hyaluronan synthesis in bone marrow mesenchymal progenitor cells: predominant expression of HAS1 mRNA and up-regulated hyaluronan synthesis in bone marrow cells derived from multiple myeloma patients. Blood, The Journal of the American Society of Hematology, (2002), 100(7), 2578-2585.

[21]. Fraser, J. R. E., Laurent, T. C., & Laurent, U. B. G. Hyaluronan: its nature, distribution, functions and turnover. Journal of internal medicine, (1997), 242(1), 27-33.

**[22].** Tsanaktsidou, E., Kammona, O., & Kiparissides, C. Recent developments in hyaluronic acid-based hydrogels for cartilage tissue engineering applications. Polymers, (2022), 14(4), 839.

[23]. Yang, H., Song, L., Zou, Y., Sun, D., Wang, L., Yu, Z., & Guo, J.. Role of hyaluronic acids and potential as regenerative biomaterials in wound healing. ACS Applied Bio Materials, (2020), 4(1), 311-324.

**[24].** Water, J. J., Schack, M. M., Velazquez-Campoy, A., Maltesen, M. J., van de Weert, M., & Jorgensen, L. Complex coacervates of hyaluronic acid and lysozyme: Effect on protein structure and physical stability. European Journal of Pharmaceutics and Biopharmaceutics, (2014), 88(2), 325-331.

[25]. De Rosa, M., D'agostino, A., La Gatta, A., & Schiraldi, C. (2019). U.S. Patent No. 10,266,611. Washington, DC: U.S. Patent and Trademark Office.

[26]. Edsman, K., Nord, L. I., Ohrlund, Å., Lärkner, H., & Kenne, A. H. Gel properties of hyaluronic acid dermal fillers. Dermatologic Surgery, (2012), 38(7pt2), 1170-1179.

[27]. Schanté, C. E., Zuber, G., Herlin, C., & Varidamme, T. F. Chemical modifications of hyaluronic acid for the synthesis of derivatives for a broad range of biomedical applications. Carbohydrate polymers, (2011), 85(3), 469-489.

[28]. Aeschlimann, D., & Bulpitt, P. (2007). U.S. Patent No. 7,196,180. Washington, DC: U.S. Patent and Trademark Office.

[29]. Njikang, G. N., Yu, X., Liu, F., & Manesis, N. J. (2022). U.S. Patent Application No. 17/396,597.

[30]. Lebreton, P., & Guetta, O. (2022). U.S. Patent No. 11,260,015. Washington, DC: U.S. Patent and Trademark Office.

[31]. Guillen, K. H., & Tezel, A. (2021). U.S. Patent No. 10,905,797. Washington, DC: U.S. Patent and Trademark Office.

[32]. Wortzman, M. S., Narins, R., & Lin, X. (2013). U.S. Patent No. 8,455,459. Washington, DC: U.S. Patent and Trademark Office.

[33]. Stroumpoulis, D., Mudd, C. S., & Tezel, A. (2013). U.S. Patent No. 8,394,782. Washington, DC: U.S. Patent and Trademark Office.

[34]. Tranchepain, F., Brunel, F., Bigand, V., & Vincent, S. (2023). U.S. Patent Application No. 18/022,344.

[35].Tauzin, B. V. (2017). U.S. Patent No. 9,782,490. Washington, DC: U.S. Patent and Trademark Office.

[36]. Njikang, G. N., Liu, F., Yu, X., Manesis, N. J., & Paliwal, S. (2016). U.S. Patent No. 9,408,797. Washington, DC: U.S. Patent and Trademark Office.

[37]. Paliwal, S., Njikang, G. N., Yu, X., Liu, F., & Manesis, N. J. (2014).

[38]. Njikang, G. N., Yu, X., Liu, F., & Manesis, N. J. (2013). U.S. Patent Application No. 13/706,221.

[39]. Tomihata, K., & Ikada, Y. (1997). Preparation of cross-linked hyaluronic acid films of low water content. Biomaterials, 18(3), 189-195.

**[40].** Karlsson, M., & Edsman, K. (2019). U.S. Patent Application No. 16/111,493.

[41]. Woodward, J., Ranjit-Reeves, R., Katz, D. F., Bernardini, F. P., & Fagien, S. Comparing Water Absorption of Food and Drug Administration-Approved Hyaluronic Acid Fillers. Dermatologic Surgery, (2021), 47(9), 1237-1242.

**[42].** Shope, C., Andrews, L., Eason, C., & Schlesinger, T. Juvéderm: What we know now. Dermatological Reviews, (2023), 4(2), 90-95.

**[43].** Barral, G. (2022). U.S. Patent Application No. 17/604,587.

**[44].** Ramli, H., Zainal, N. F. A., Hess, M., & Chan, C. H. Basic principle and good practices of rheology for polymers for teachers and beginners. Chemistry Teacher International, (2022), 4(4), 307-326.

**[45].** Ebara, M., Kotsuchibashi, Y., Narain, R., Idota, N., Kim, Y. J., Hoffman, J. M., ... & Aoyagi, T. (2014). Smart biomaterials. Springer.

## Claims

**1.** A method for preparing a cross-linked hyaluronic acid gel comprising the steps:

- cross-linking hyaluronic acid with a cross-linking agent to obtain cross-linked hyaluronic acid;
- adding the cross-linked hyaluronic acid to an alcohol to obtain a first reaction mixture;
- adding the first reaction mixture to an alkaline alcoholic solution to obtain a second reaction mixture;
- separating at least one sediment from the first reaction mixture and/or at least one sediment from the second reaction mixture;
- treating the combined sediments with a buffer having a phosphate buffer to obtain the cross-linked hyaluronic acid gel.

**2.** The method according to claim 1, wherein the hyaluronic acid has a weight average molecular weight from 1,000,000 to 3,000,000 g/mol measured by gel permeation chromatography.

**3.** The method according to claim 1 or 2, wherein the crosslinking agent comprises at least two epoxy-groups.

**4.** The method according to any of the preceding claims, wherein the crosslinking agent is 1,4-butanediol diglycidyl ether.

5. The method according to any of the preceding claims, wherein the amount of the crosslinking agent is such that the degree of transverse connections in the cross-linked hyaluronic acid is from 0.05 to 0.3.

6. The method according to any of the preceding claims, wherein the crosslinking is performed under alkaline conditions.

7. The method according to any of the preceding claims, wherein the alcohol is ethanol.

8. The method according to any of the preceding claims, wherein the alkaline alcoholic solution is a solution of NaOH in ethanol.

9. The method according to any of the preceding claims, wherein the method comprises a further step of drying the combined sediments before treating the combined sediments with the buffer.

10. The method according to any of the preceding claims, wherein a weight ratio of hyaluronic acid to cross-linking agent in the cross-linking step is from 100:1 to 20:1.

11. The method according to any of the preceding claims, wherein treating the combined sediments with a buffer having a phosphate buffer to obtain the cross-linked hyaluronic acid gel is performed with a concentration of cross-linked hyaluronic acid in the buffer from 16 to 28 mg/ml.

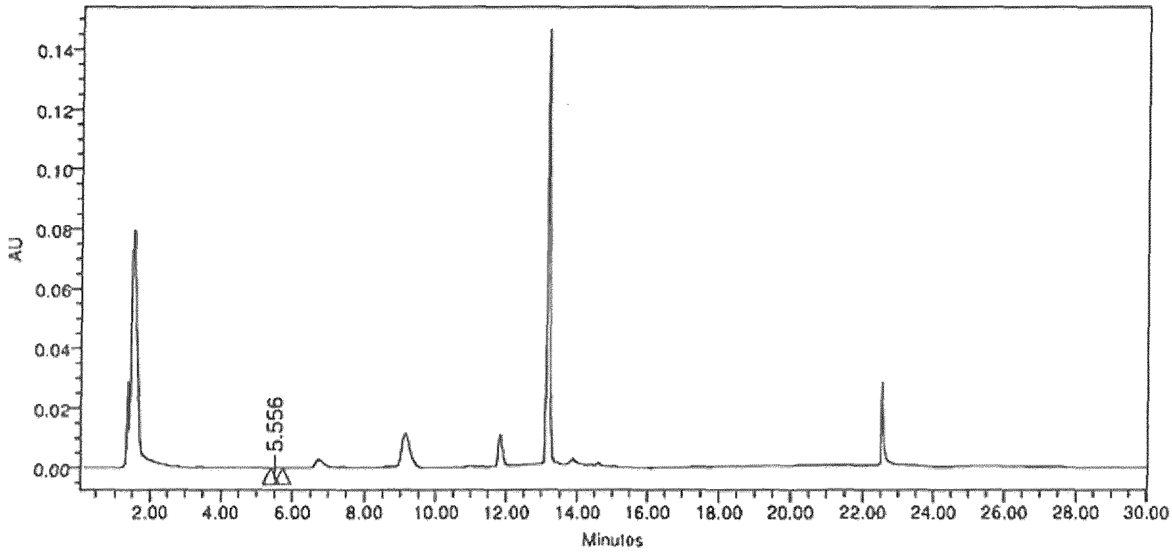


**Peak Results**

| | Name | RT | Area | Height | Amount | Units |
|---|---|---|---|---|---|---|
| 1 | Blank | 5.556 | 258 | 35 | N.A | N.A |

Fig. 1

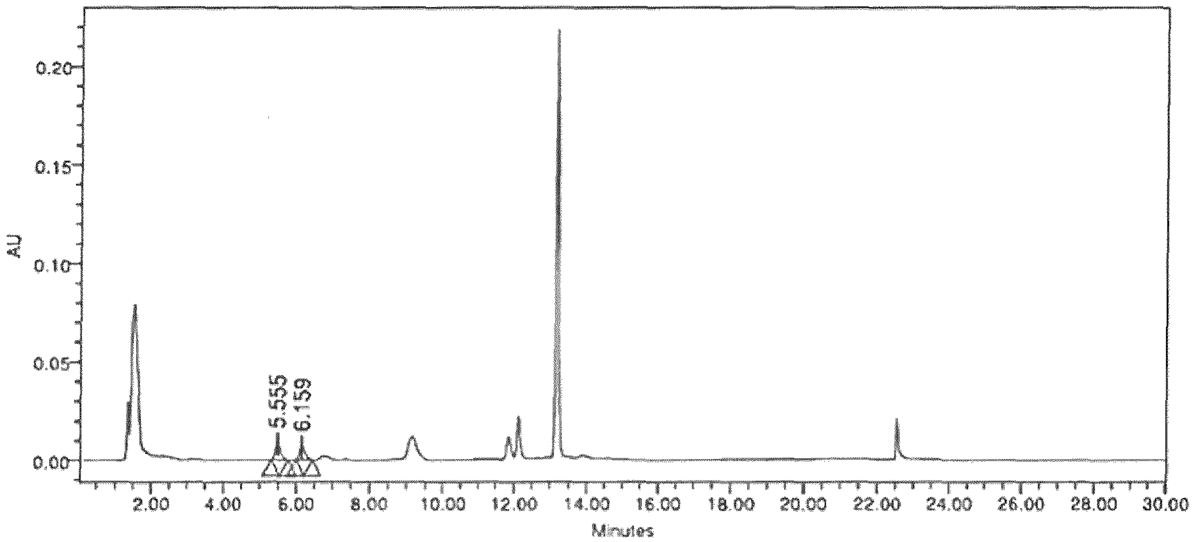


**Peak Results**

| | Name | RT | Area | Height | Amount | Units |
|---|---|---|---|---|---|---|
| 1 | BDDE | 5.555 | 85011 | 7962 | 2.0 | mg/Lit |
| 2 | ECH | 6.159 | 73032 | 6442 | 3.3 | mg/Lit |

Fig. 2

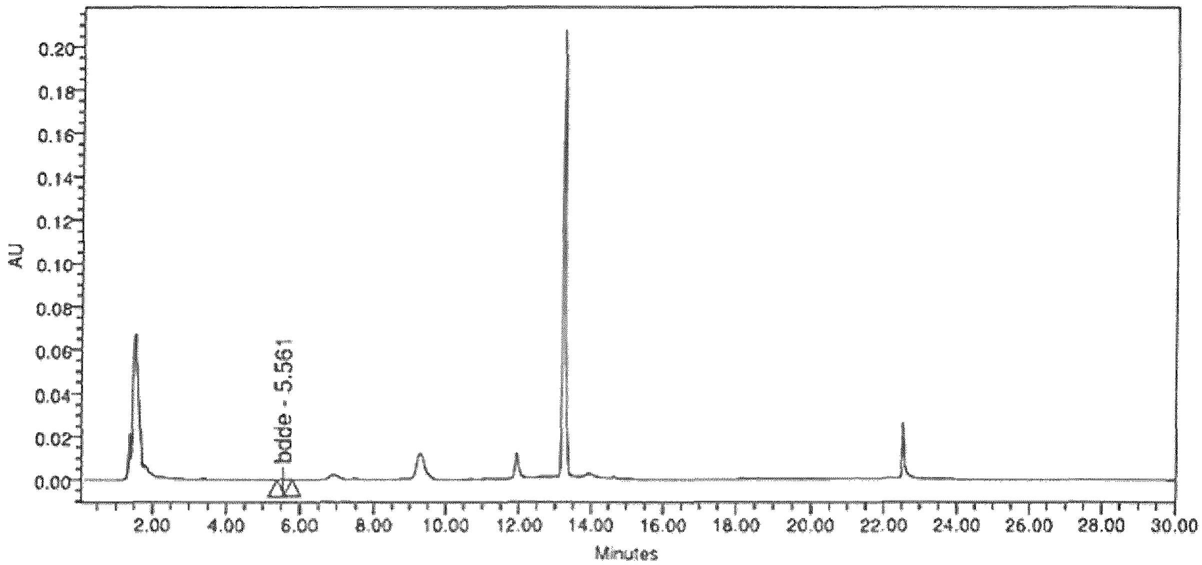


**Peak Results**

| | Name | RT | Area | Height | Amount | Units |
|---|---|---|---|---|---|---|
| 1 | bdde | 5.561 | 962 | 92 | 0.010 | mg/Lit |

Fig. 3

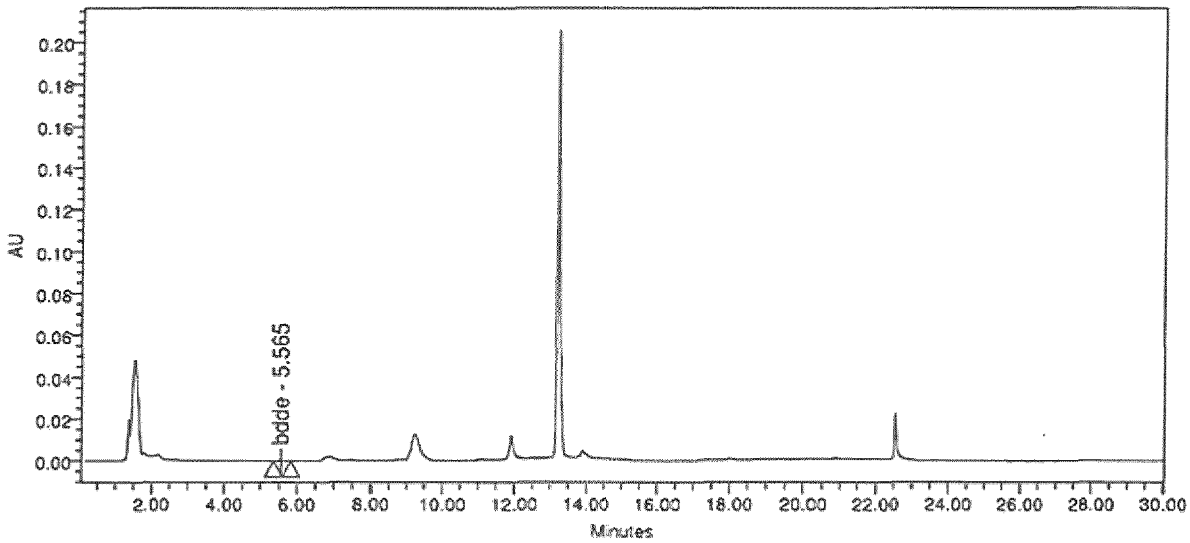


**Peak Results**

| | Name | RT | Area | Height | Amount | Units |
|---|---|---|---|---|---|---|
| 1 | bdde | 5.565 | 1559 | 131 | 0.020 | mg/Lit |

Fig. 4

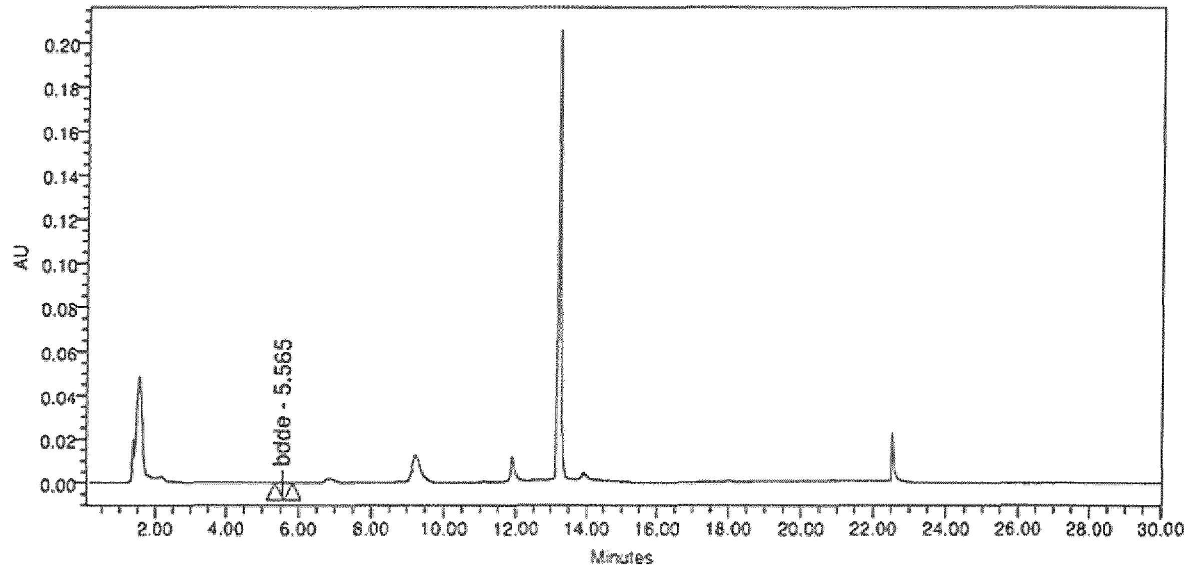


**Peak Results**

| | Name | RT | Area | Height | Amount | Units |
|---|---|---|---|---|---|---|
| 1 | bdde | 5.565 | 1559 | 131 | 0.020 | mg/Lit |

Fig. 5

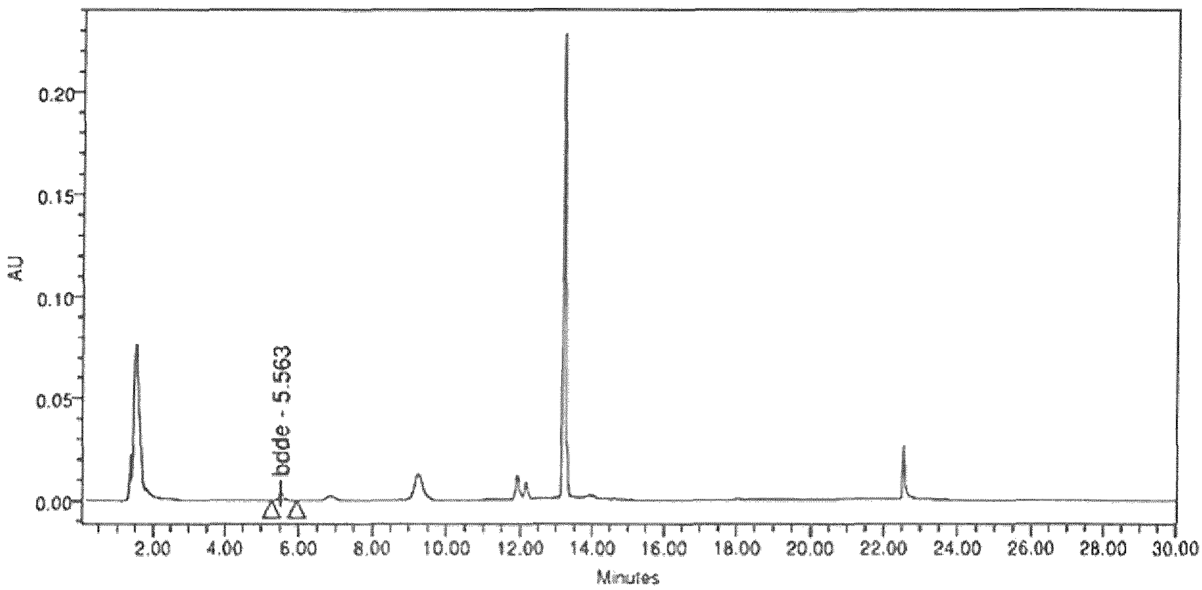


**Peak Results**

| | Name | RT | Area | Height | Amount | Units |
|---|---|---|---|---|---|---|
| 1 | bdde | 5.563 | 34512 | 3123 | 1.000 | mg/Lit |

Fig. 6

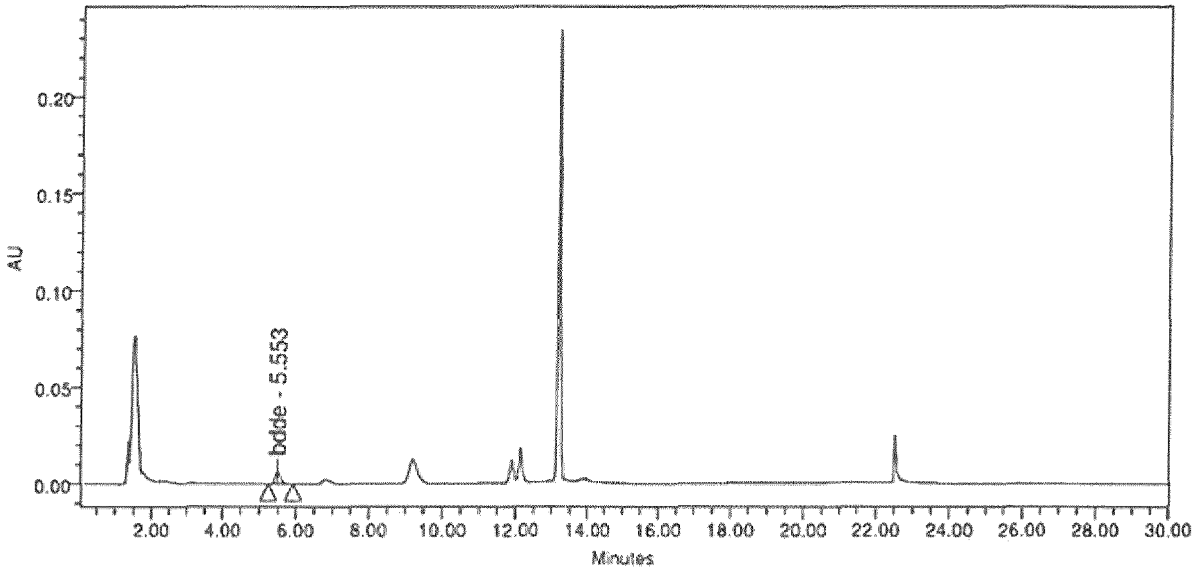


**Peak Results**

| | Name | RT | Area | Height | Amount | Units |
|---|---|---|---|---|---|---|
| 1 | bdde | 5.553 | 67732 | 6229 | 2.000 | mg/Lit |

Fig. 7

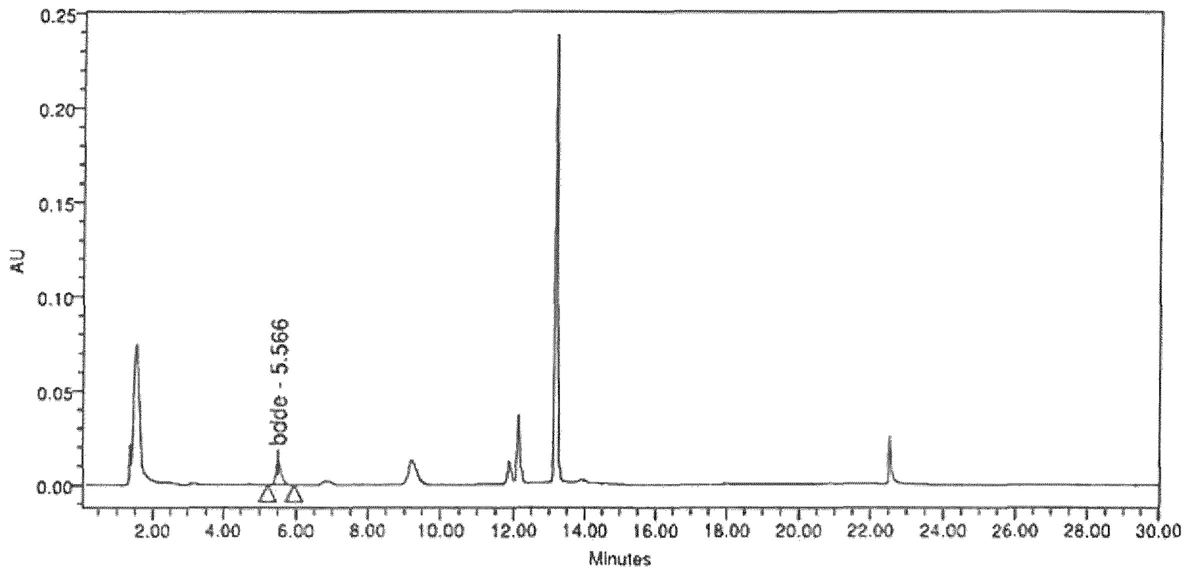


**Peak Results**

| | Name | RT | Area | Height | Amount | Units |
|---|---|---|---|---|---|---|
| 1 | bdde | 5.566 | 132018 | 12176 | 4.000 | mg/Lit |

Fig. 8

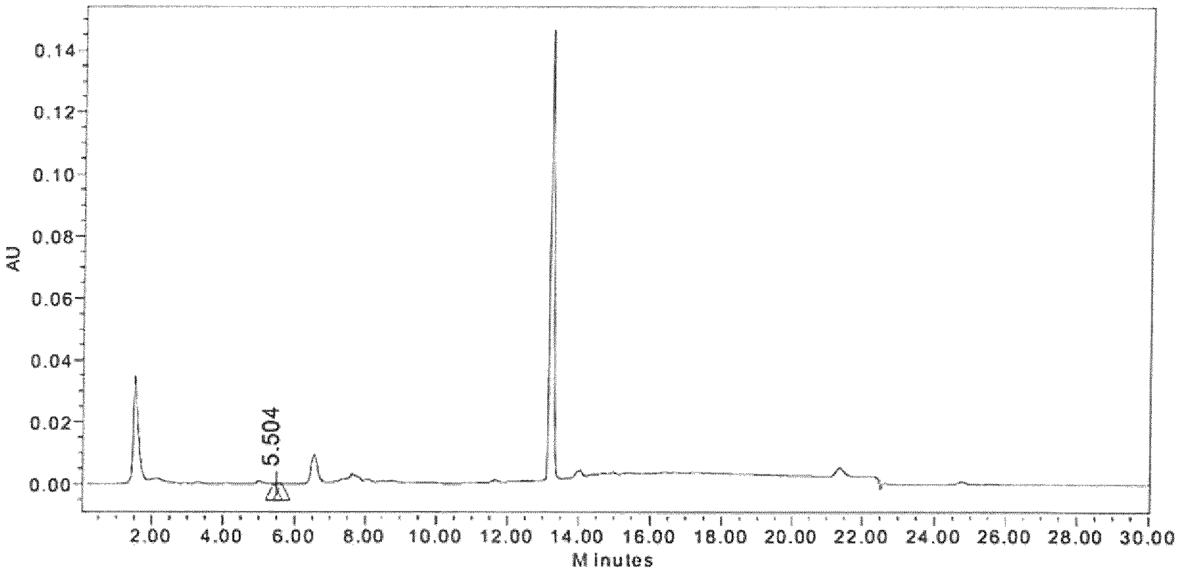


**Peak Results**

| | Name | RT | Area | Height | Amount | Units |
|---|---|---|---|---|---|---|
| 1 | BDDE | 5.504 | 207 | 25 | N.A | mg/Lit |

Fig. 9

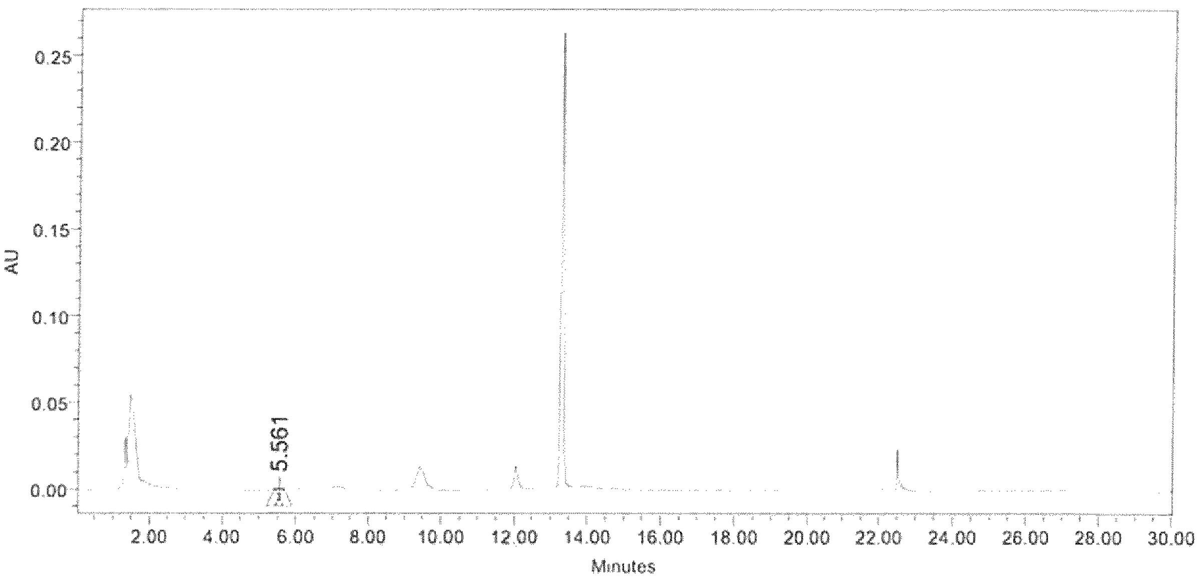


**Peak Results**

| | Name | RT | Area | Height | Amount | Units |
|---|---|---|---|---|---|---|
| 1 | BDDE | 5.561 | 1617 | 199 | N.A | mg/Lit |

Fig. 10

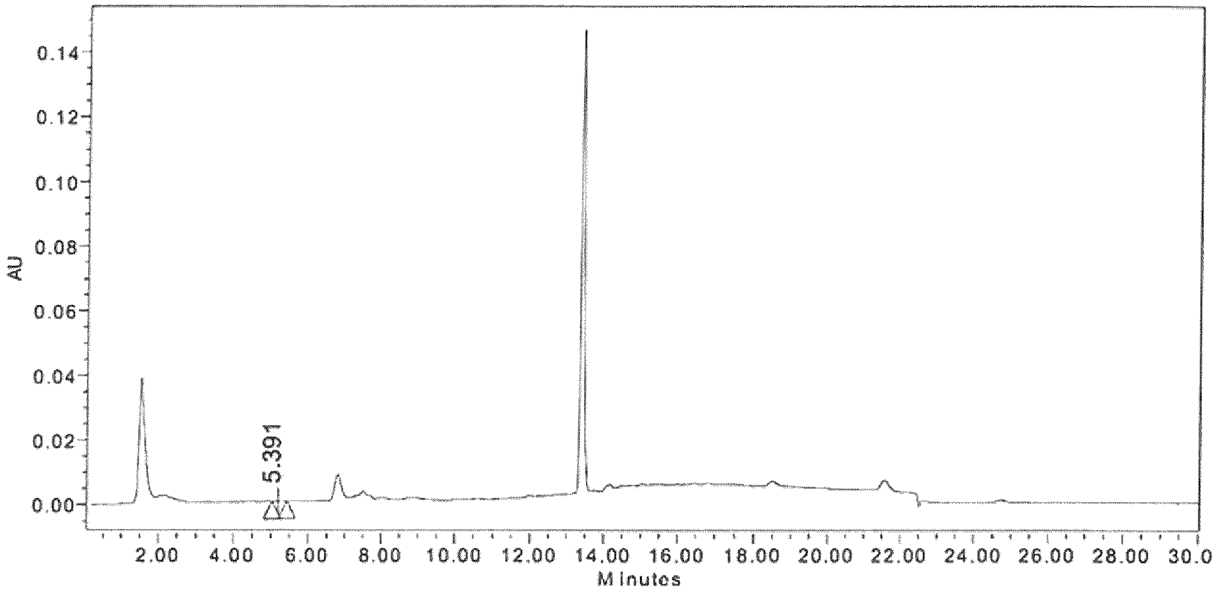


**Peak Results**

| | Name | RT | Area | Height | Amount | Units |
|---|---|---|---|---|---|---|
| 1 | BDDE | 5.391 | 1450 | 125 | N.A. | mg/Lit |

Fig. 11

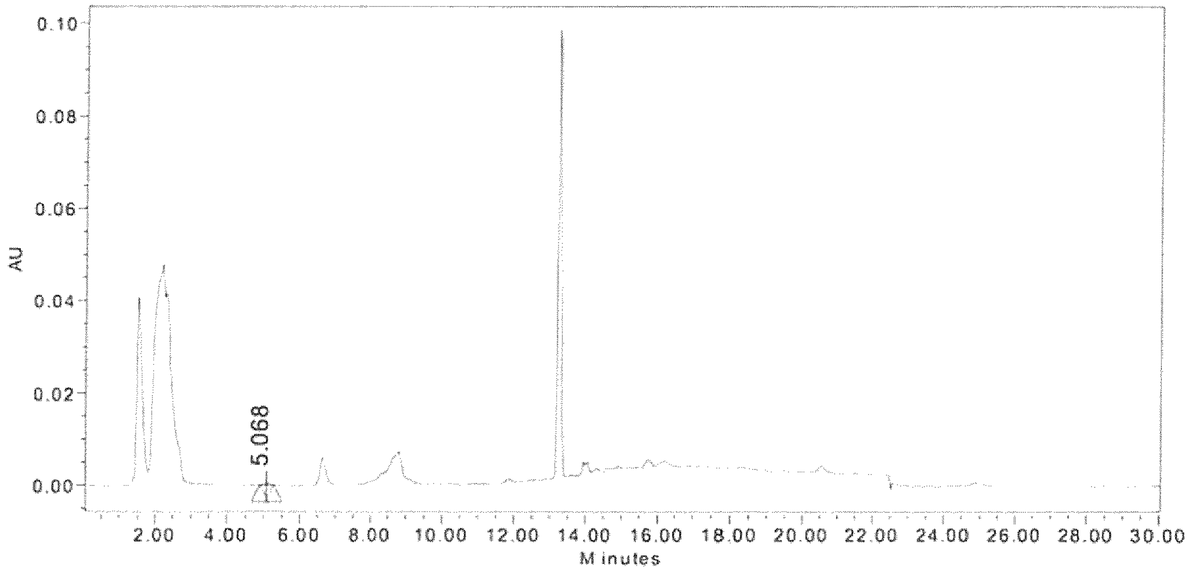


**Peak Results**

| | Name | RT | Area | Height | Amount | Units |
|---|---|---|---|---|---|---|
| 1 | BDDE | 5.268 | 9685 | 1089 | N.A. | mg/Lit |

Fig. 12

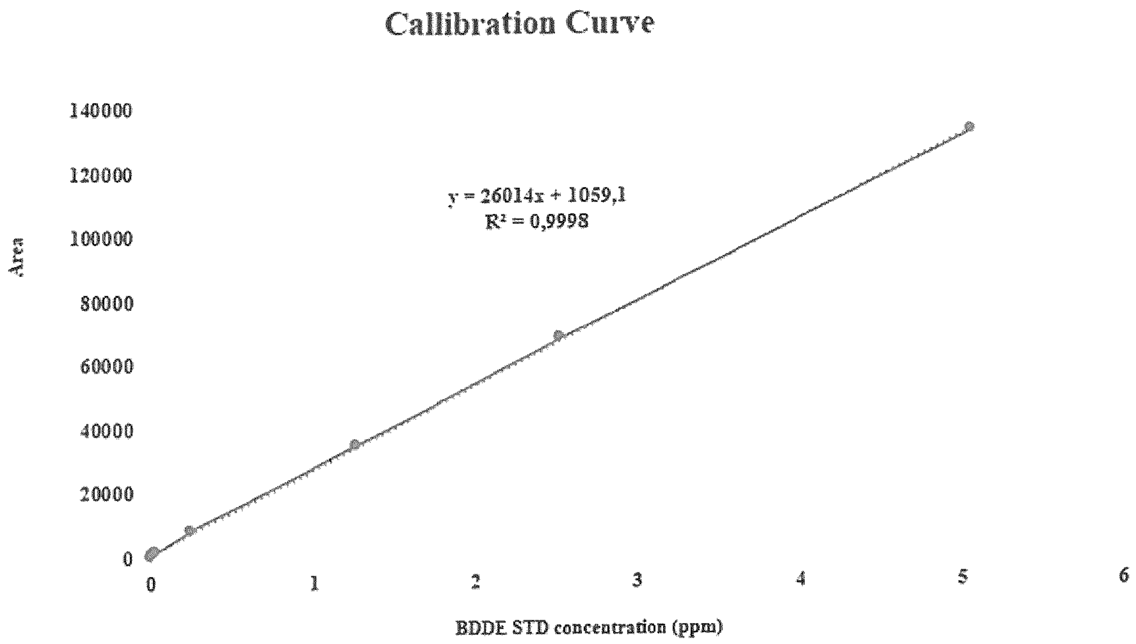
Callibration Curve
y = 26014x + 1059,1
R² = 0,9998
Area
140000
120000
100000
80000
60000
40000
20000
0
0
1
2
3
4
5
6
BDDE STD concentration (ppm)

Fig. 13

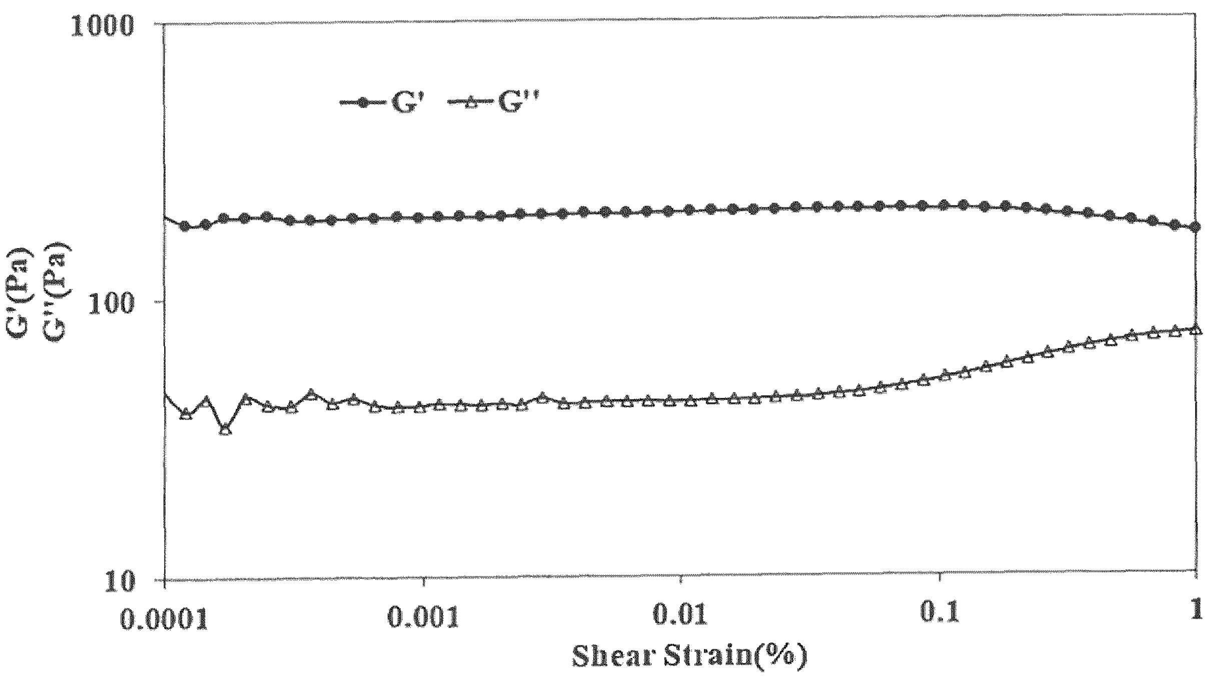
1000
100
10
G'(Pa)
G''(Pa)
G'
G''
0.0001
0.001
0.01
0.1
1
Shear Strain(%)

Fig. 14

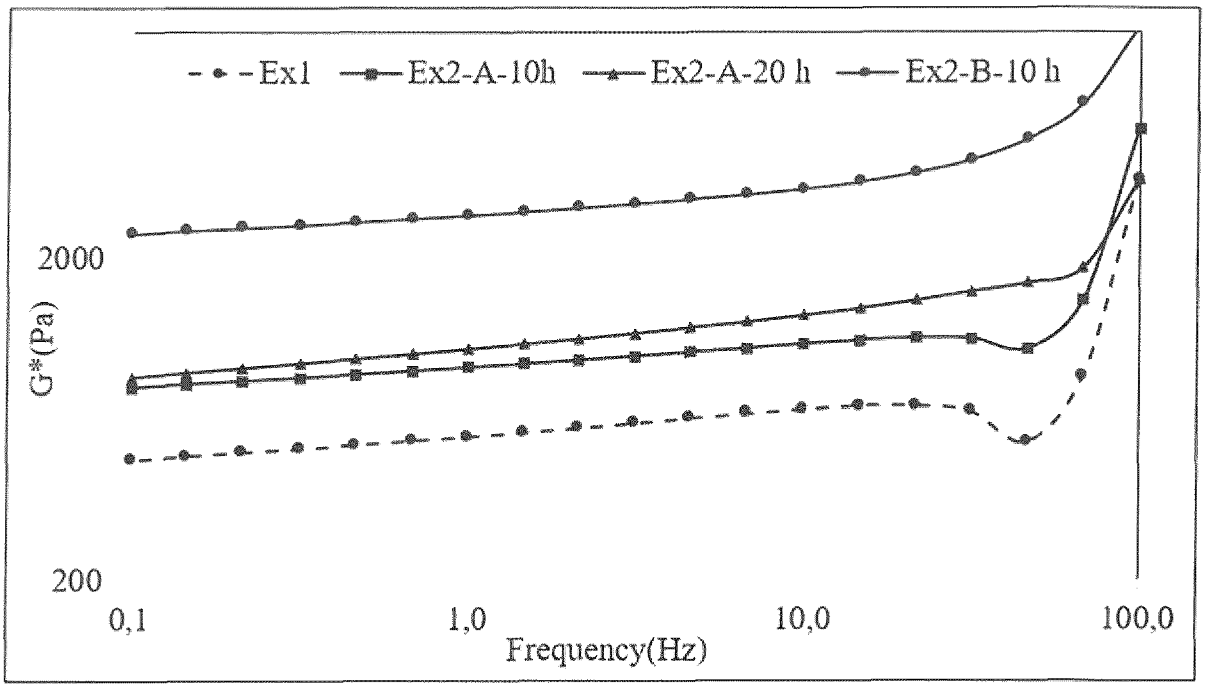
- - Ex1
Ex2-A-10h
Ex2-A-20 h
Ex2-B-10 h
2000
200
G*(Pa)
0,1
1,0
10,0
100,0
Frequency(Hz)

Fig. 15

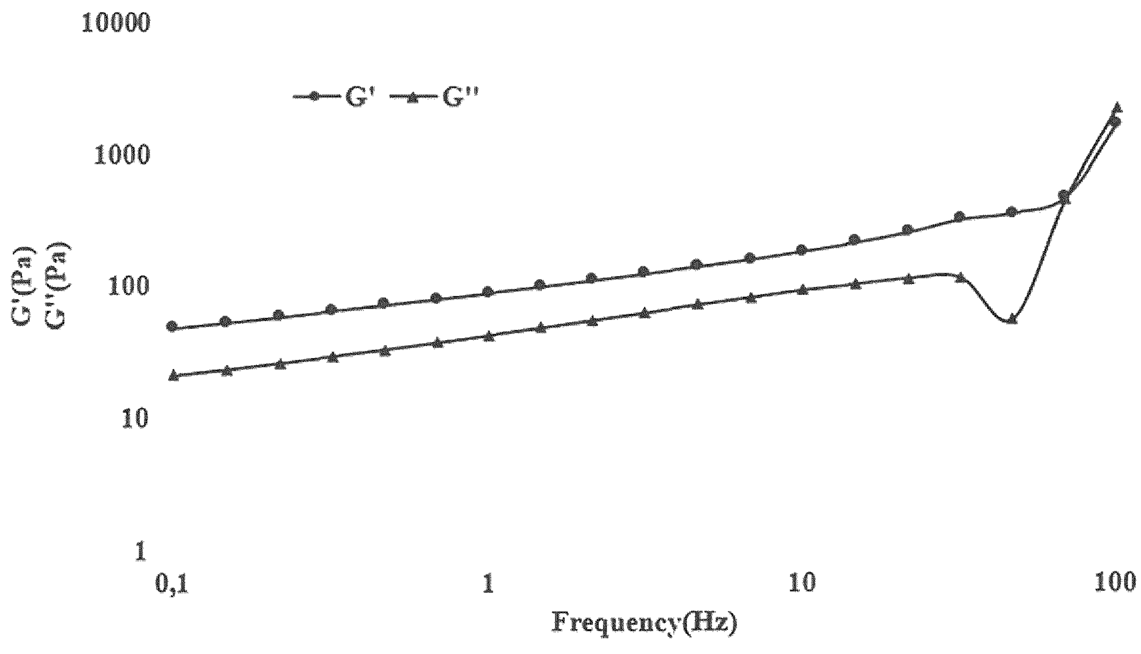
10000
1000
100
10
1
G'
G''
G'(Pa)
G''(Pa)
0,1
1
10
100
Frequency(Hz)

Fig. 16

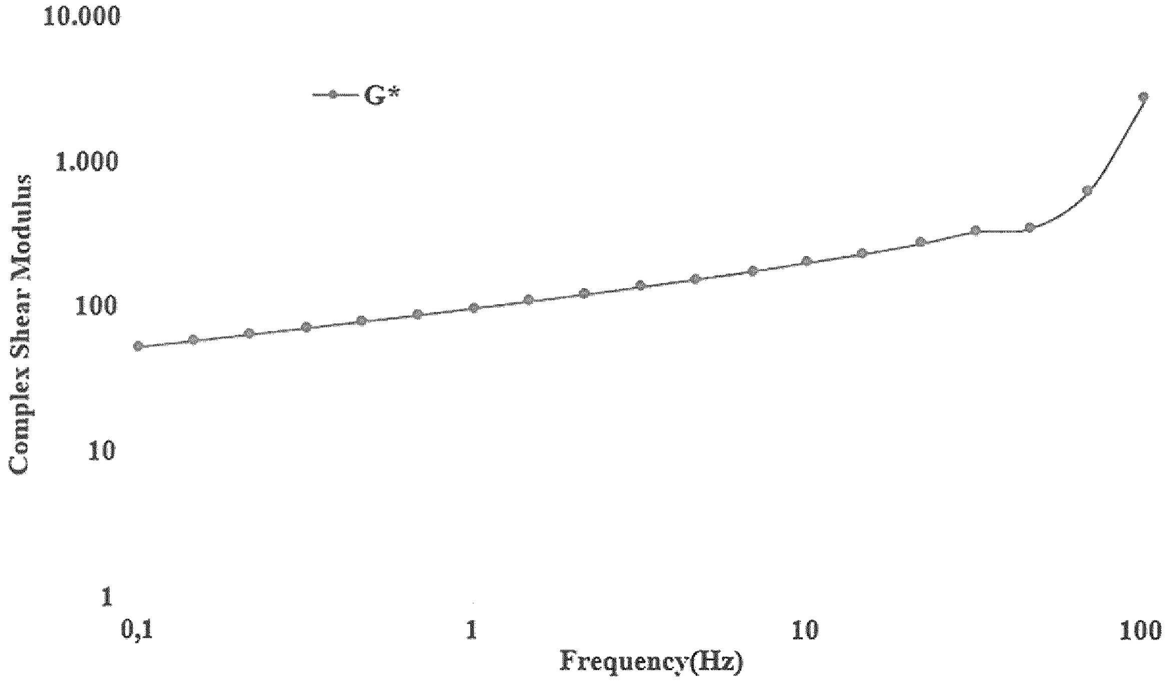
10.000
G*
1.000
Complex Shear Modulus
100
10
1
0,1
1
10
100
Frequency(Hz)

Fig. 17

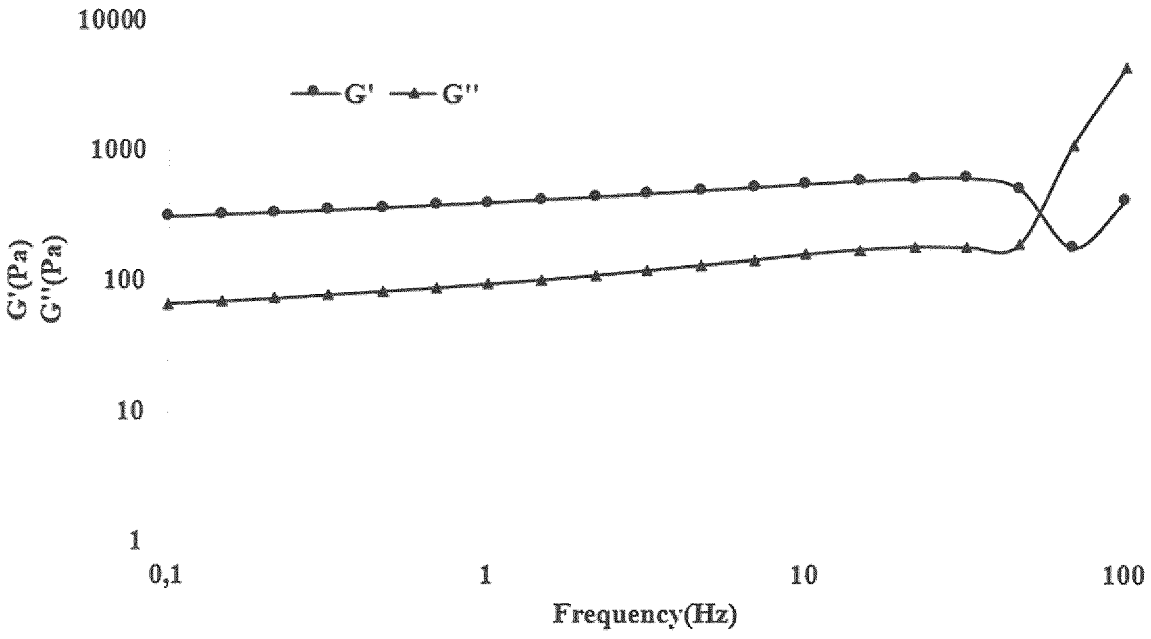
10000
G'
G''
1000
G'(Pa)
G''(Pa)
100
10
1
0,1
1
10
100
Frequency(Hz)

Fig. 18

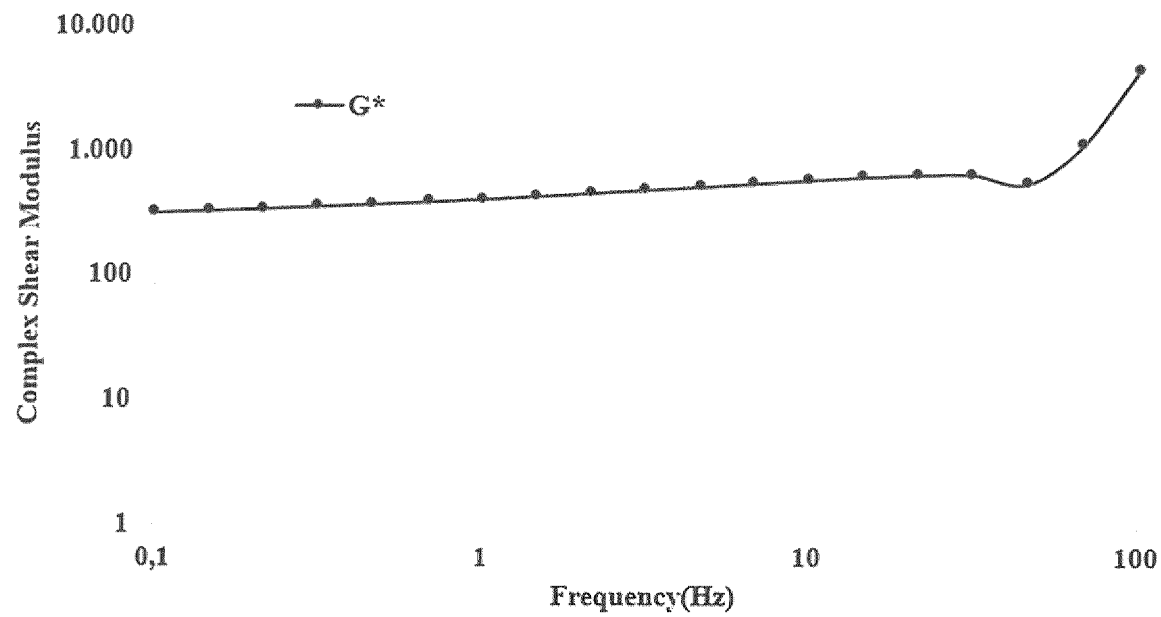
10.000
G*
1.000
100
10
1
Complex Shear Modulus
0,1
1
10
100
Frequency(Hz)

Fig. 19

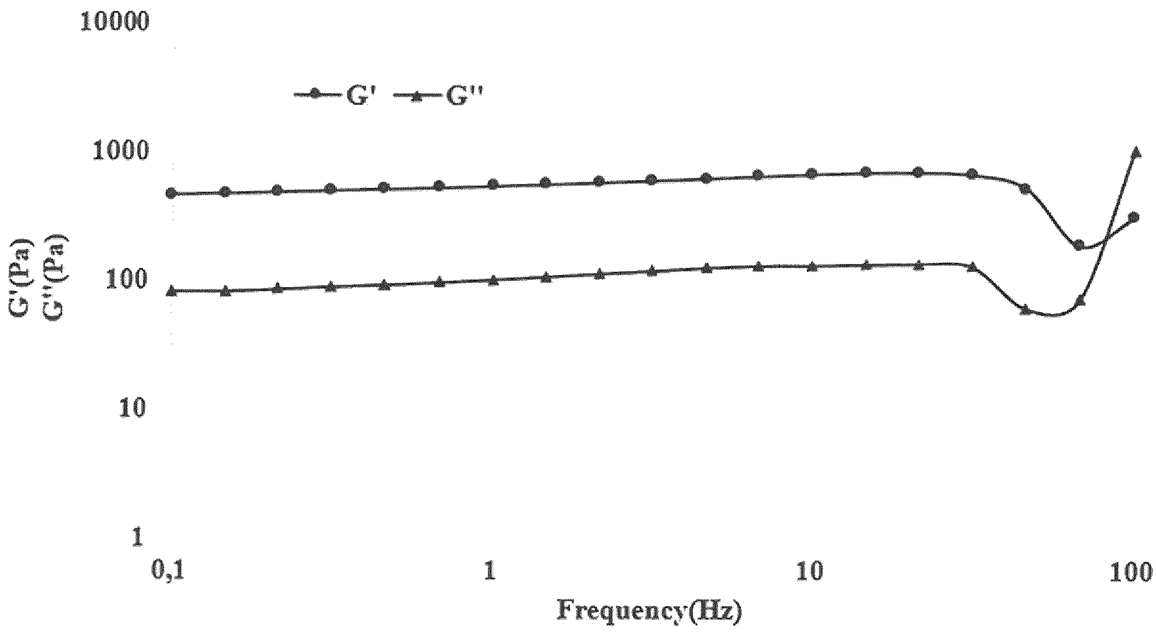
10000
G'
G''
1000
100
10
1
G'(Pa)
G''(Pa)
0,1
1
10
100
Frequency(Hz)

Fig. 20

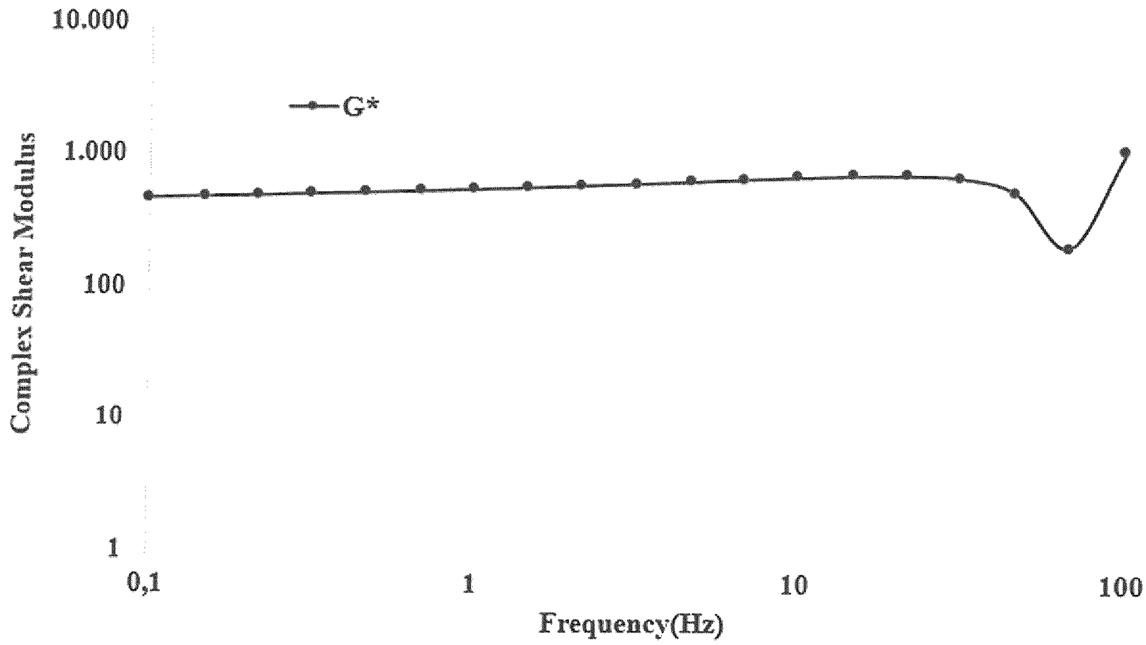
10.000
G*
1.000
Complex Shear Modulus
100
10
1
0,1
1
10
100
Frequency(Hz)

Fig. 21

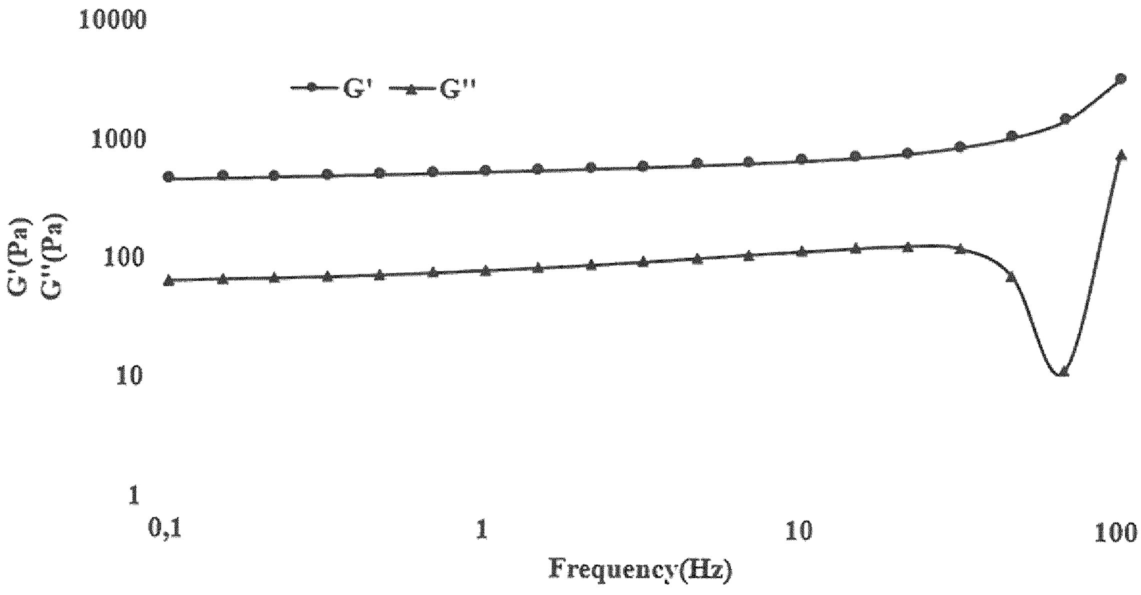
10000
G' G''
1000
G'(Pa)
G''(Pa)
100
10
1
0,1
1
10
100
Frequency(Hz)

Fig. 22

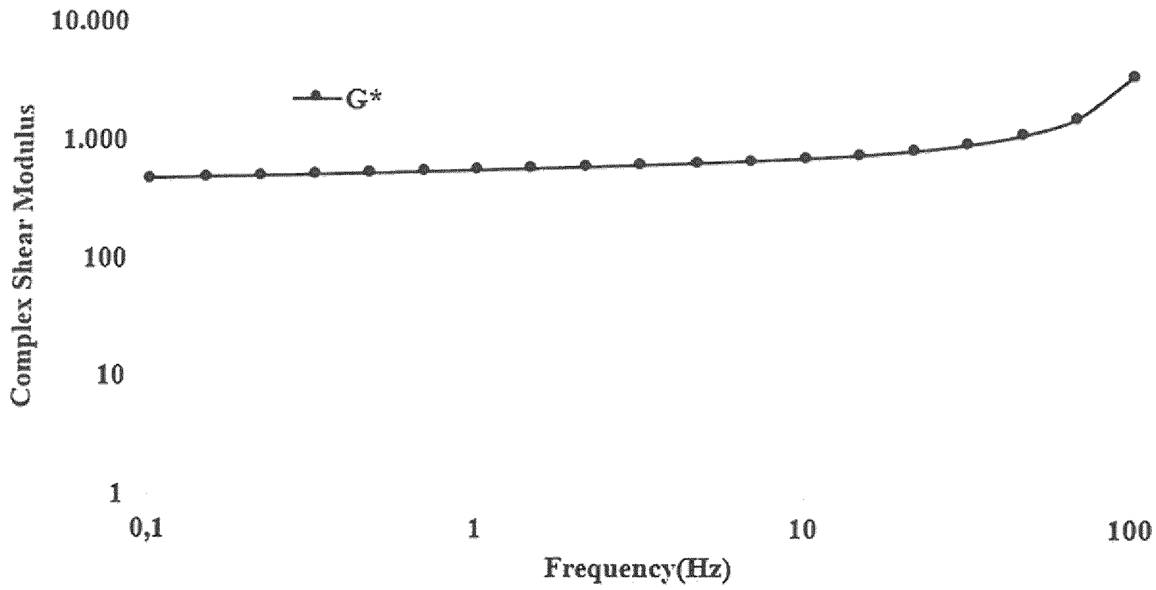
10.000
1.000
100
10
1
Complex Shear Modulus
G*
0,1
1
10
100
Frequency(Hz)

Fig. 23

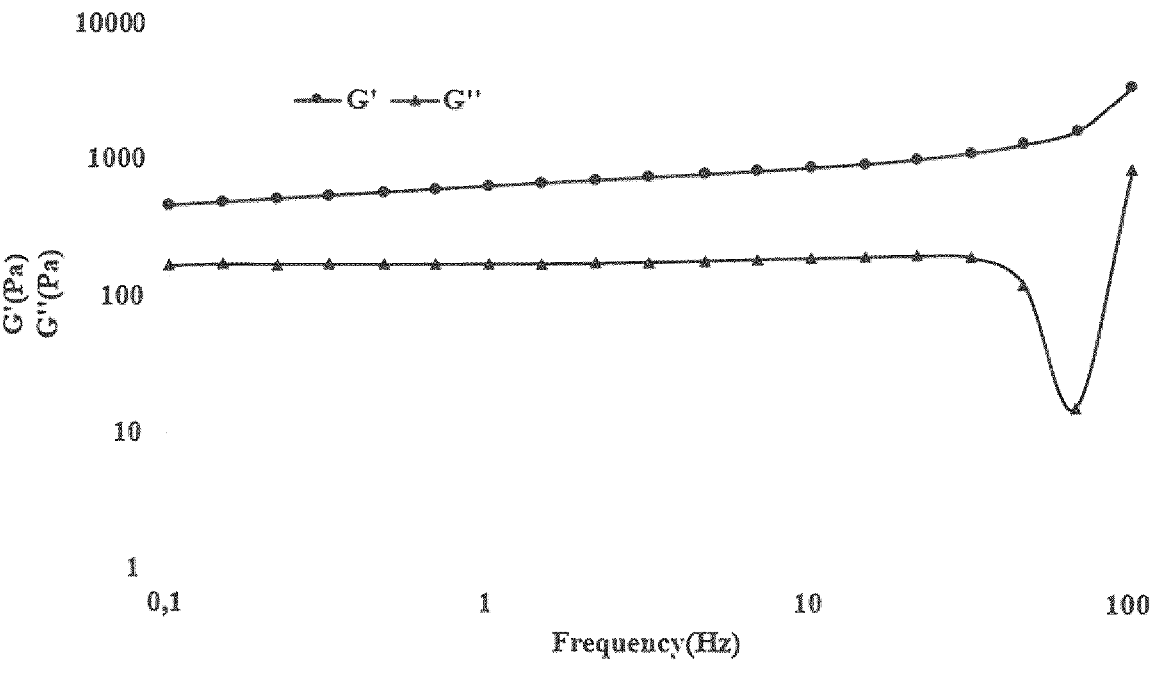
10000
1000
100
10
1
G'(Pa)
G''(Pa)
G'
G''
0,1
1
10
100
Frequency(Hz)

Fig. 24

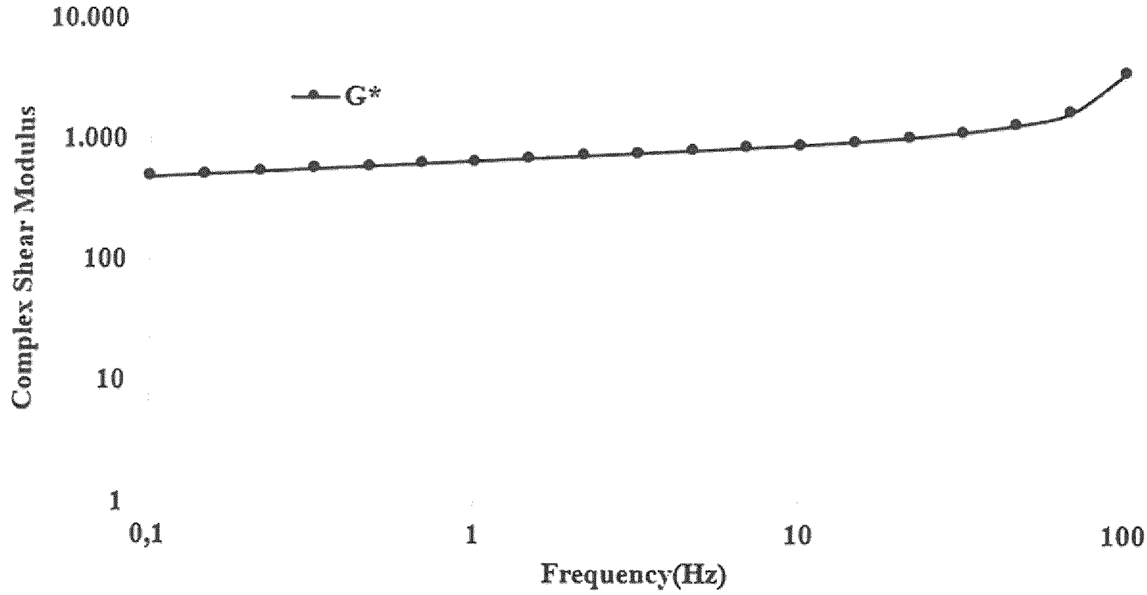
10.000
1.000
100
10
1
Complex Shear Modulus
G*
0,1
1
10
100
Frequency(Hz)

Fig.25

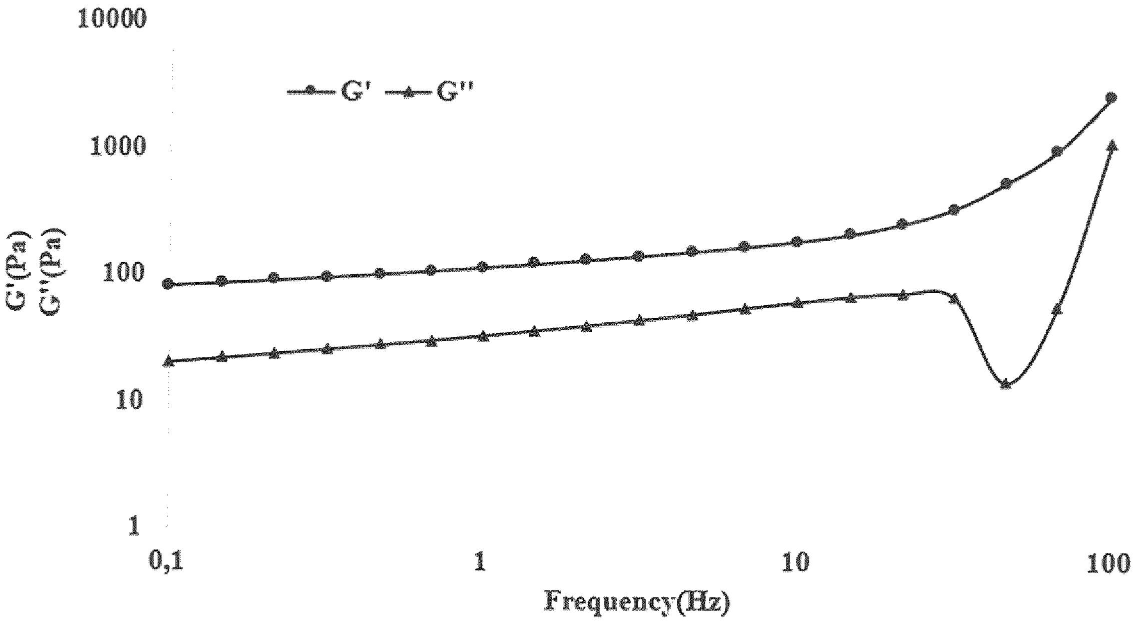
10000
1000
100
10
1
G'(Pa)
G''(Pa)
G'
G''
0,1
1
10
100
Frequency(Hz)

Fig. 26

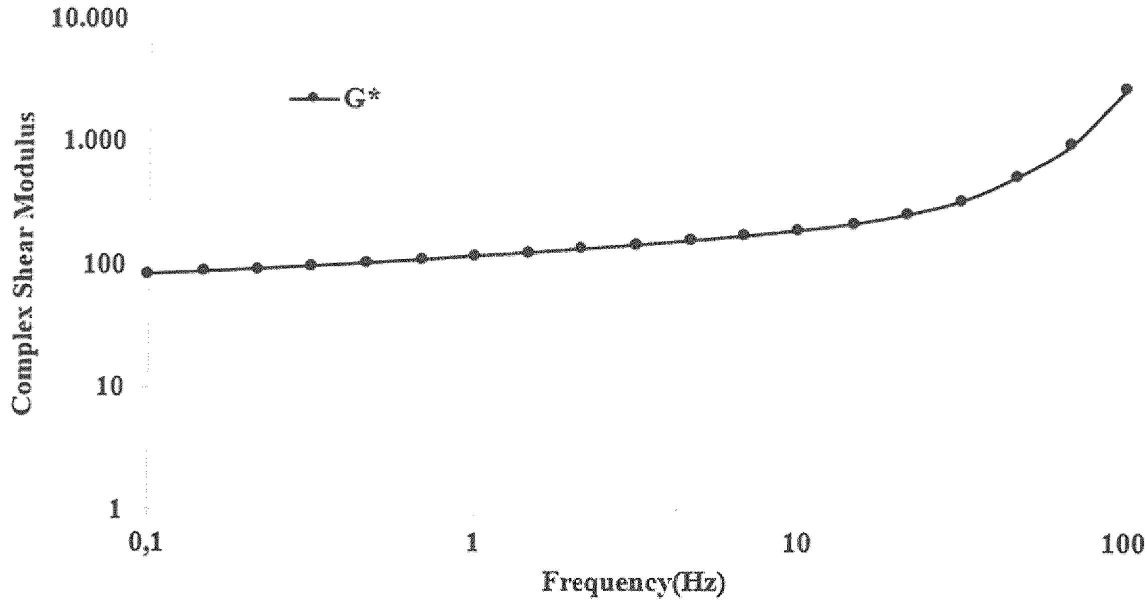
10.000
1.000
100
10
1
Complex Shear Modulus
G*
0,1
1
10
100
Frequency(Hz)

Fig. 27

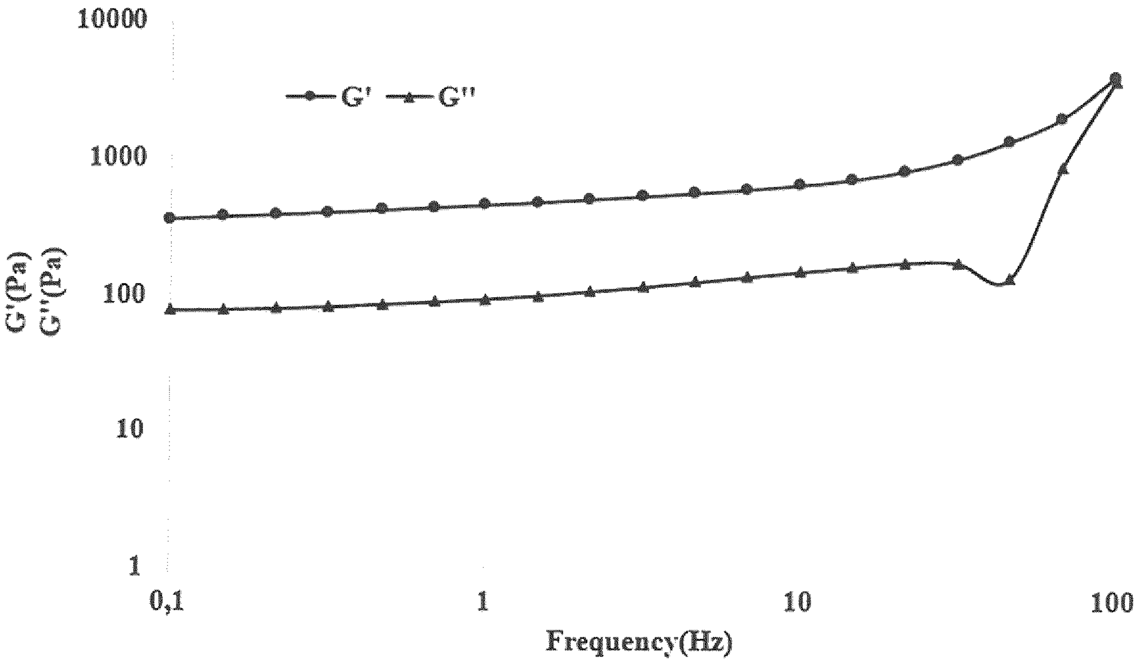
10000
1000
100
10
1
G'(Pa)
G''(Pa)
G'
G''
0,1
1
10
100
Frequency(Hz)

Fig. 28

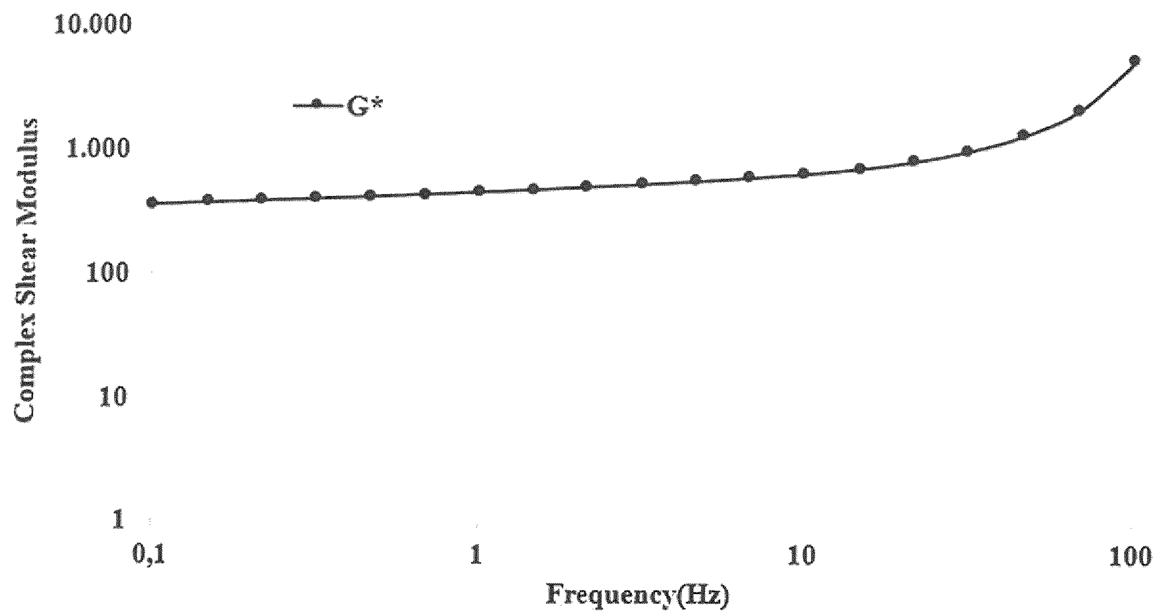
10.000
1.000
100
10
1
Complex Shear Modulus
G*
0,1
1
10
100
Frequency(Hz)

Fig. 29

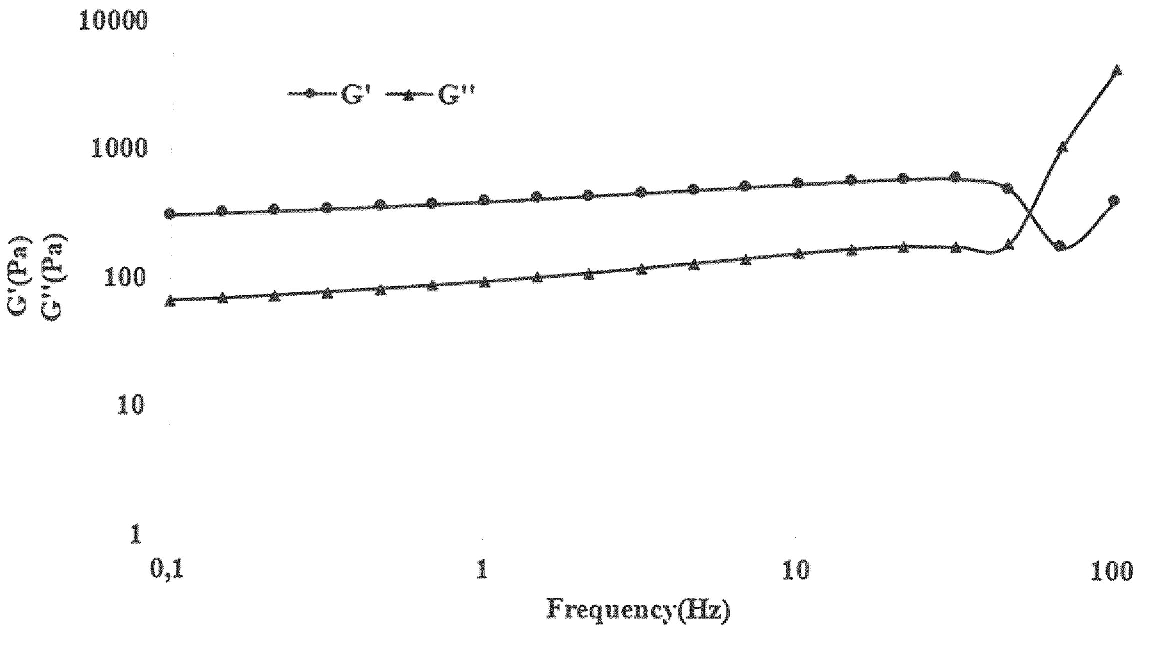
10000
1000
100
10
1
G'(Pa)
G''(Pa)
G'
G''
0,1
1
10
100
Frequency(Hz)

Fig. 30

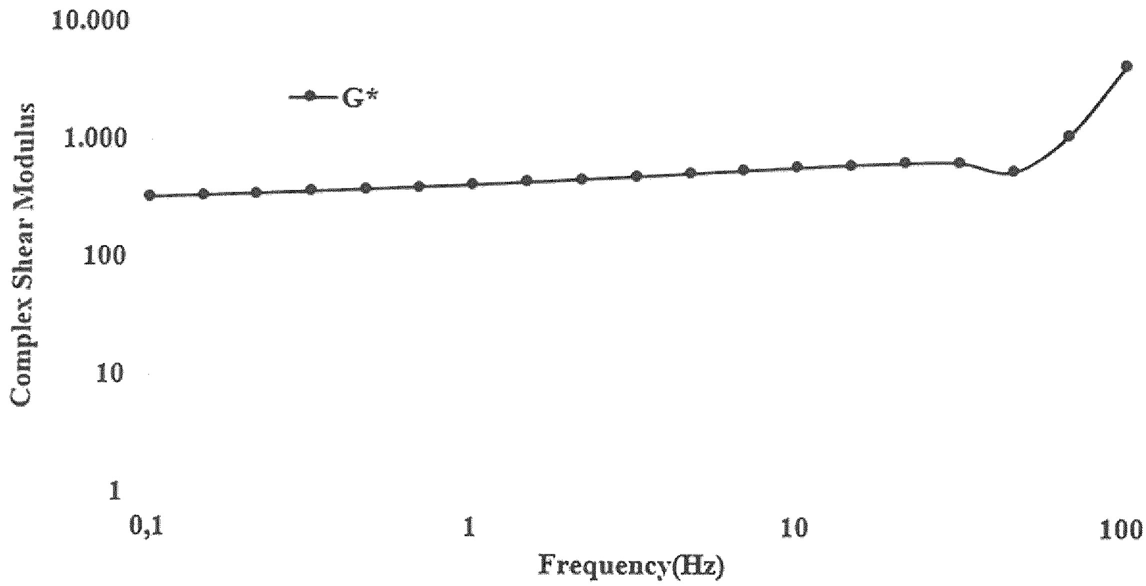
10.000
1.000
100
10
1
Complex Shear Modulus
G*
0,1
1
10
100
Frequency(Hz)

Fig. 31

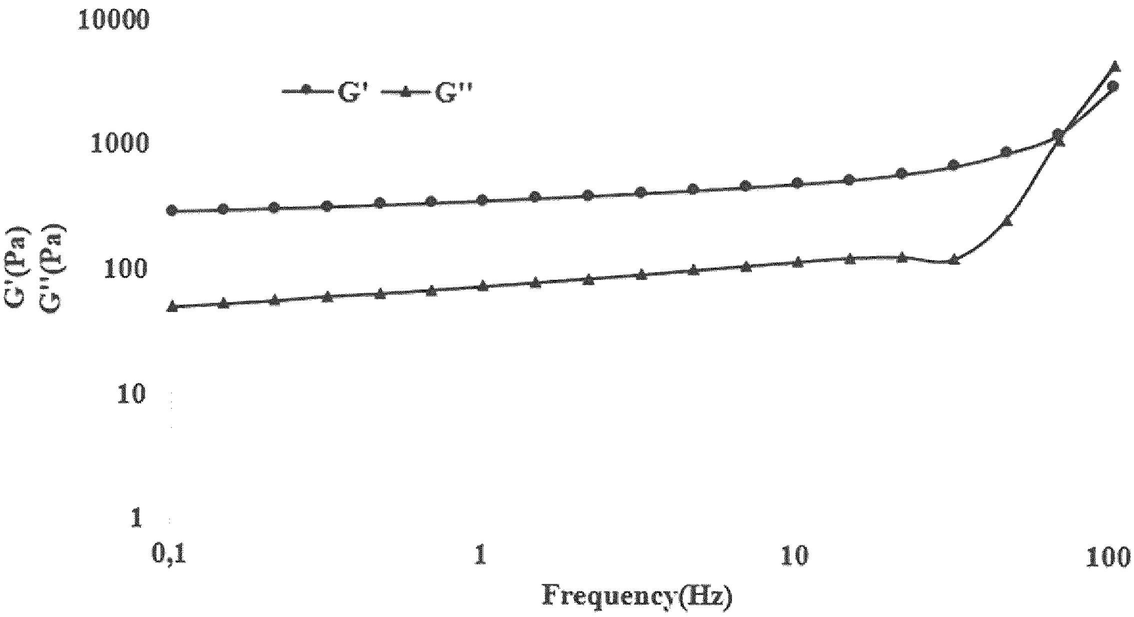
10000
1000
100
10
1
G'(Pa)
G''(Pa)
G'
G''
0,1
1
10
100
Frequency(Hz)

Fig.32

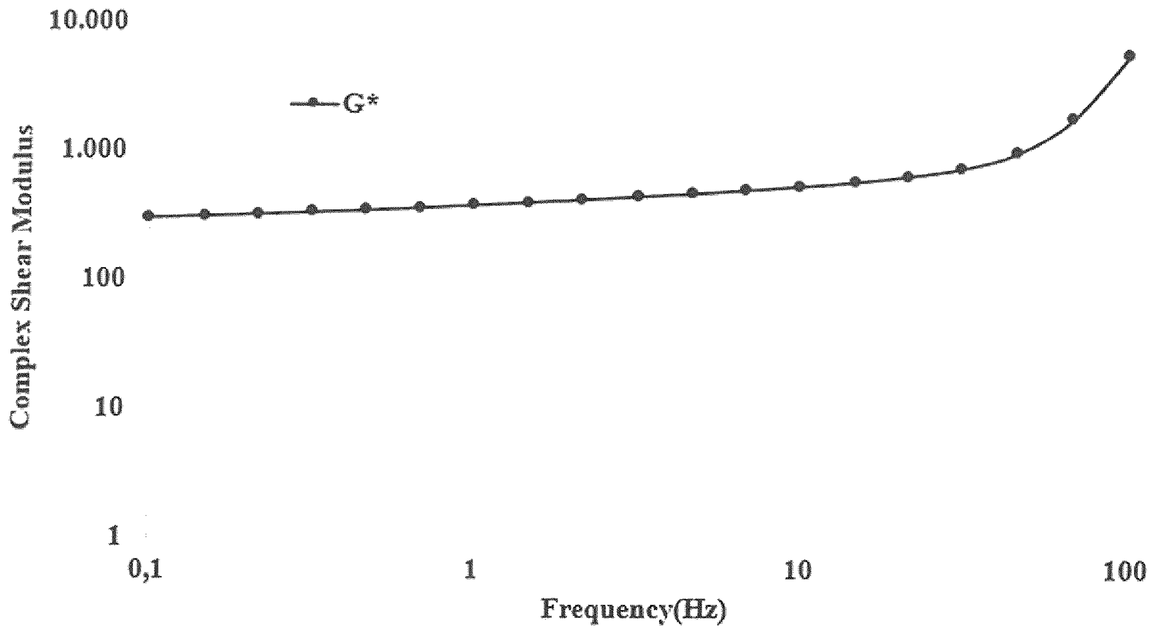
10.000
1.000
100
10
1
G*
Complex Shear Modulus
0,1
1
10
100
Frequency(Hz)

Fig. 33

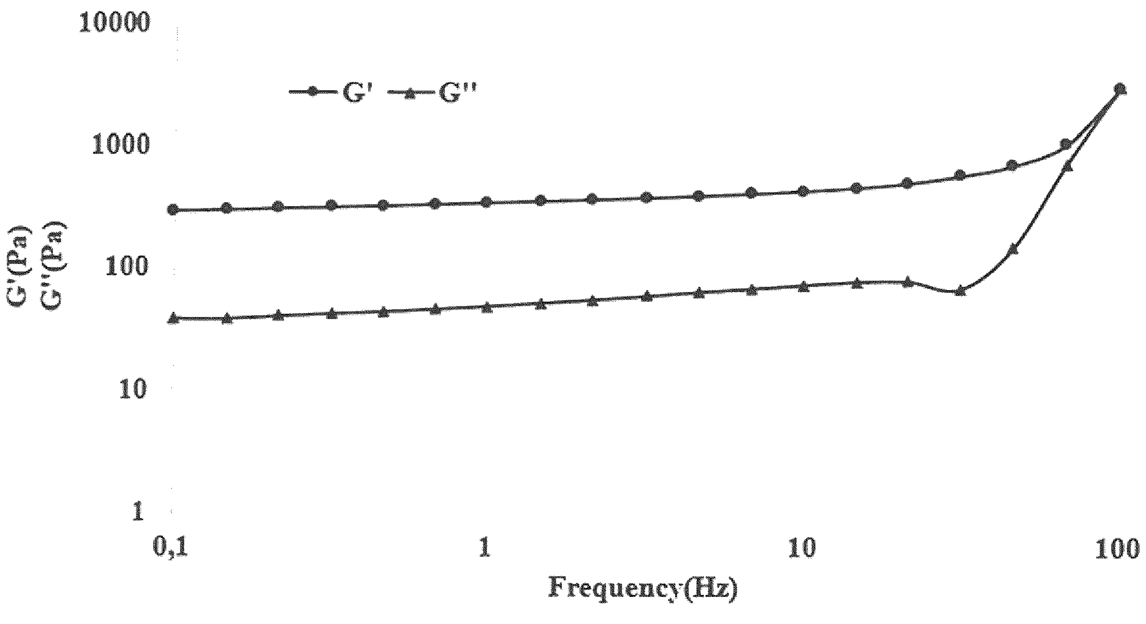
10000
1000
100
10
1
G'
G''
G'(Pa)
G''(Pa)
0,1
1
10
100
Frequency(Hz)

Fig. 34

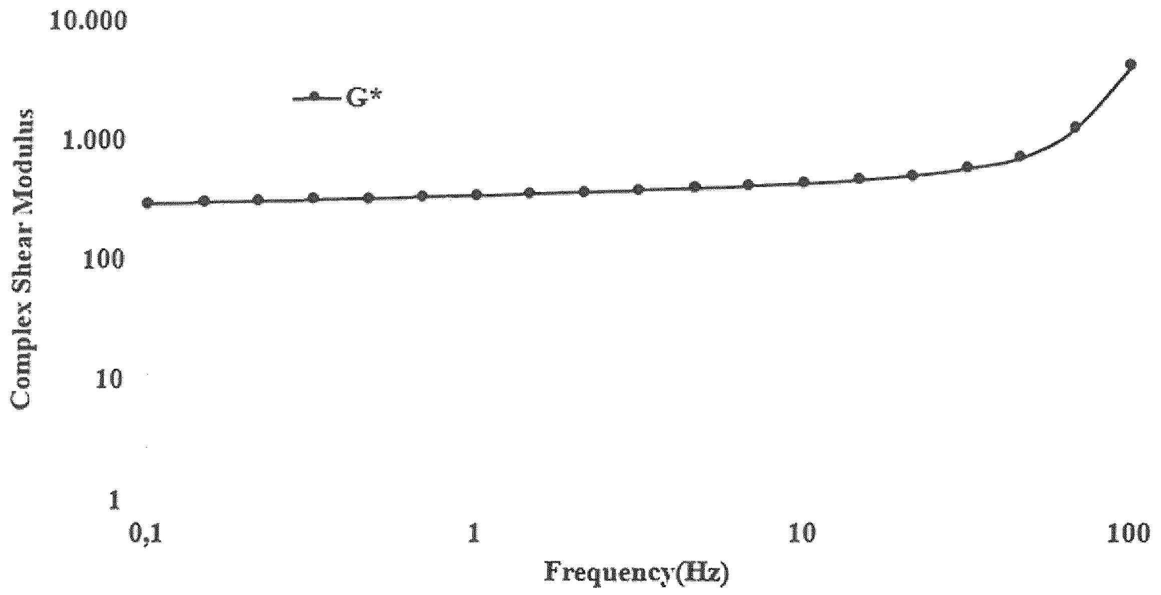
10.000
1.000
100
10
1
G*
Complex Shear Modulus
0,1
1
10
100
Frequency(Hz)

Fig. 35

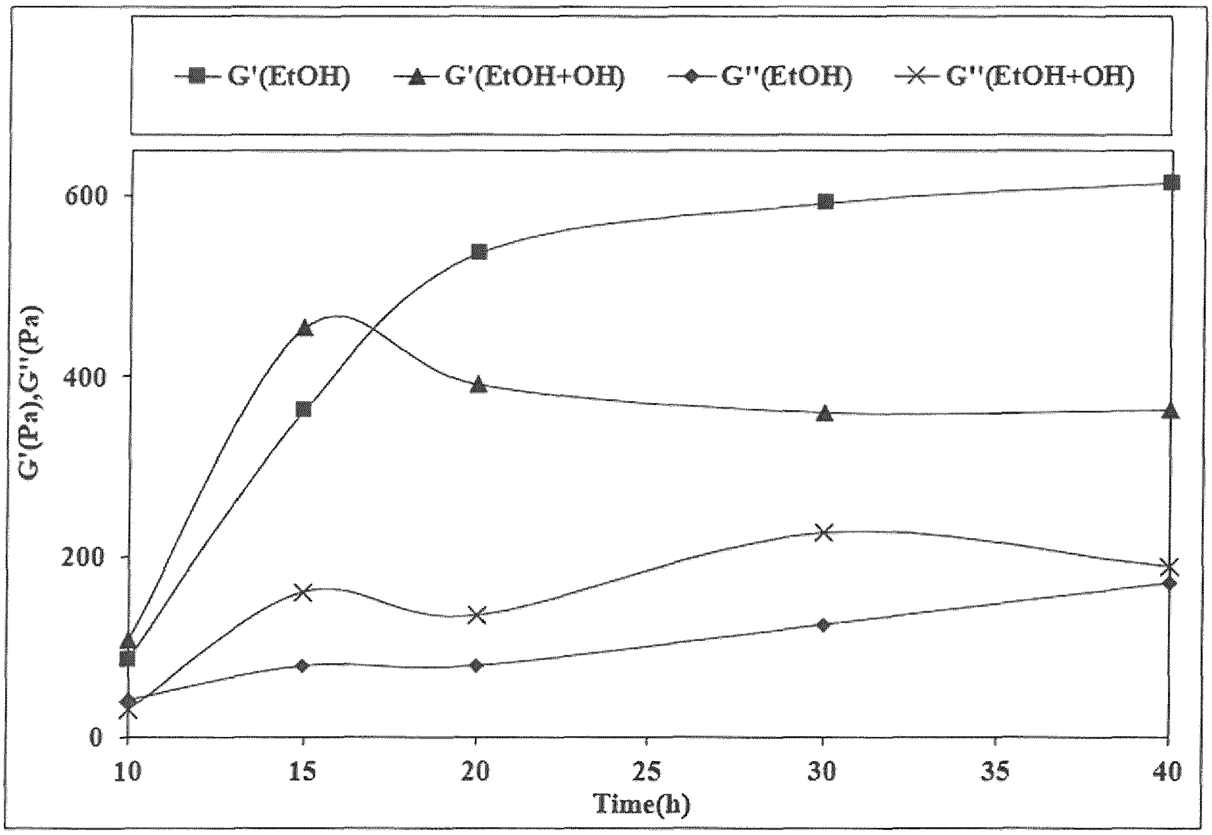
G'(EtOH)
G'(EtOH+OH)
G''(EtOH)
G''(EtOH+OH)
600
400
200
0
G'(Pa),G''(Pa)
10
15
20
25
30
35
40
Time(h)

Fig. 36

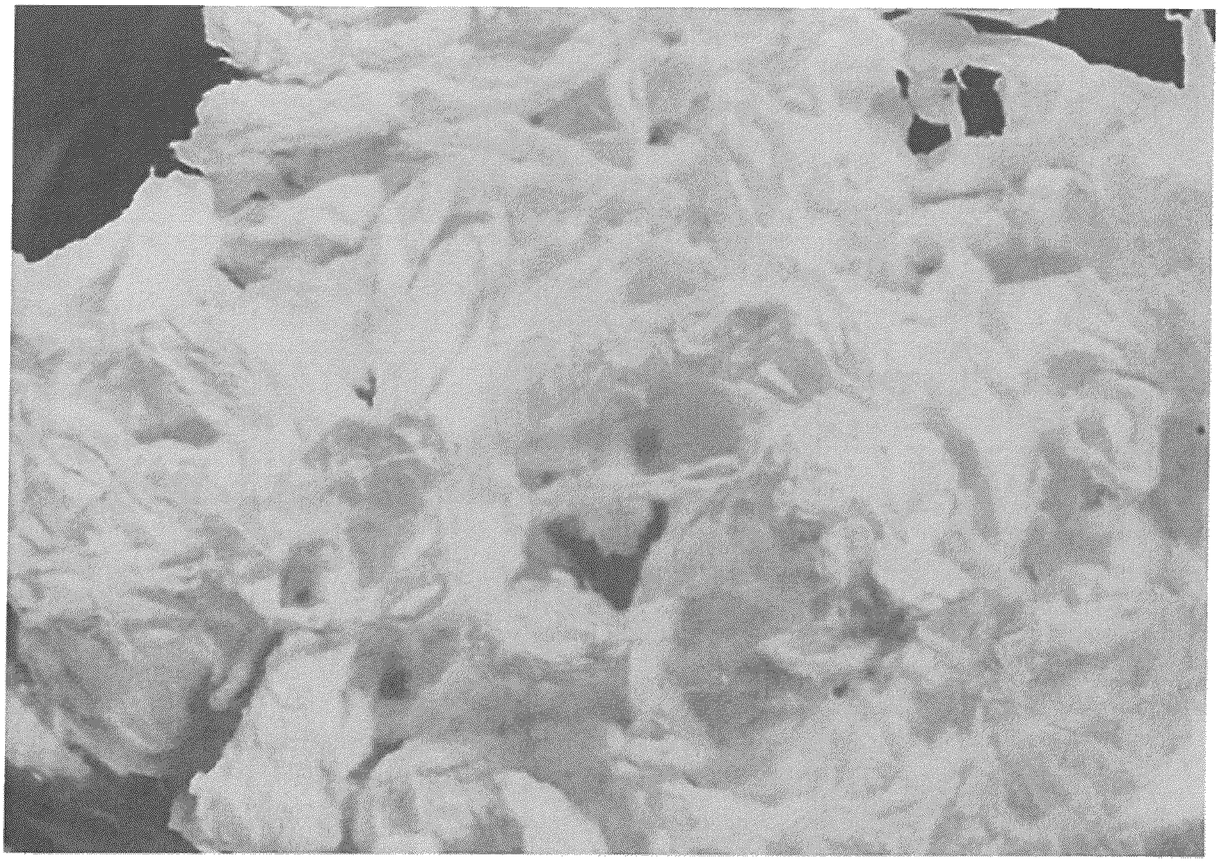

Fig. 37

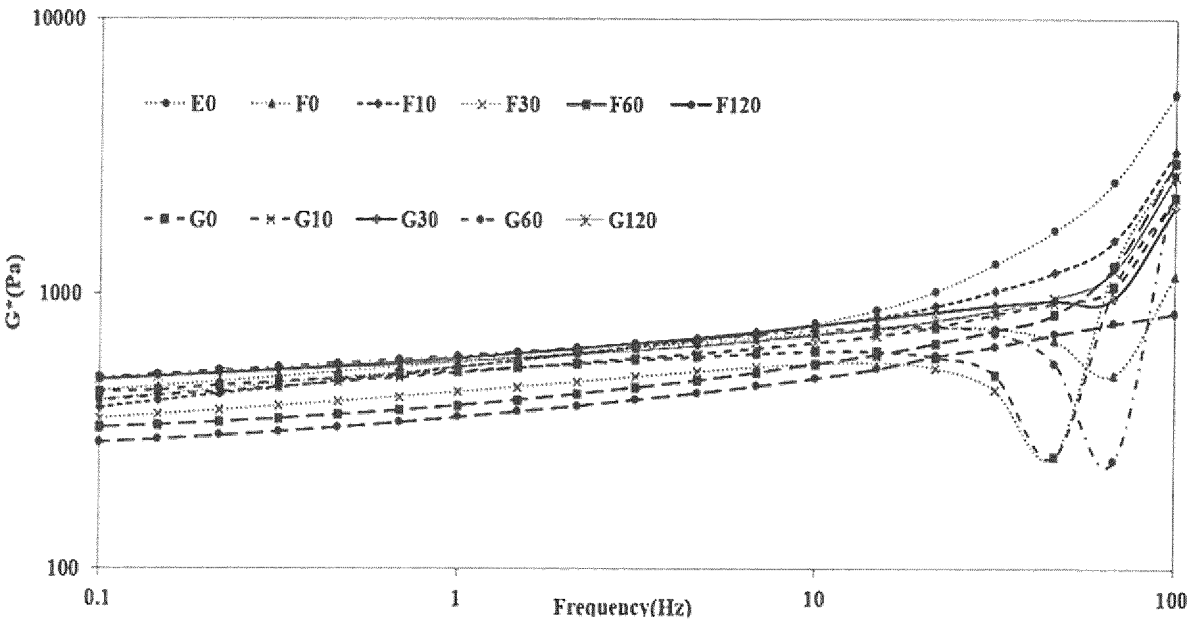
10000
1000
100
G*(Pa)
E0
F0
F10
F30
F60
F120
G0
G10
G30
G60
G120
0.1
1
10
100
Frequency(Hz)

Fig. 38

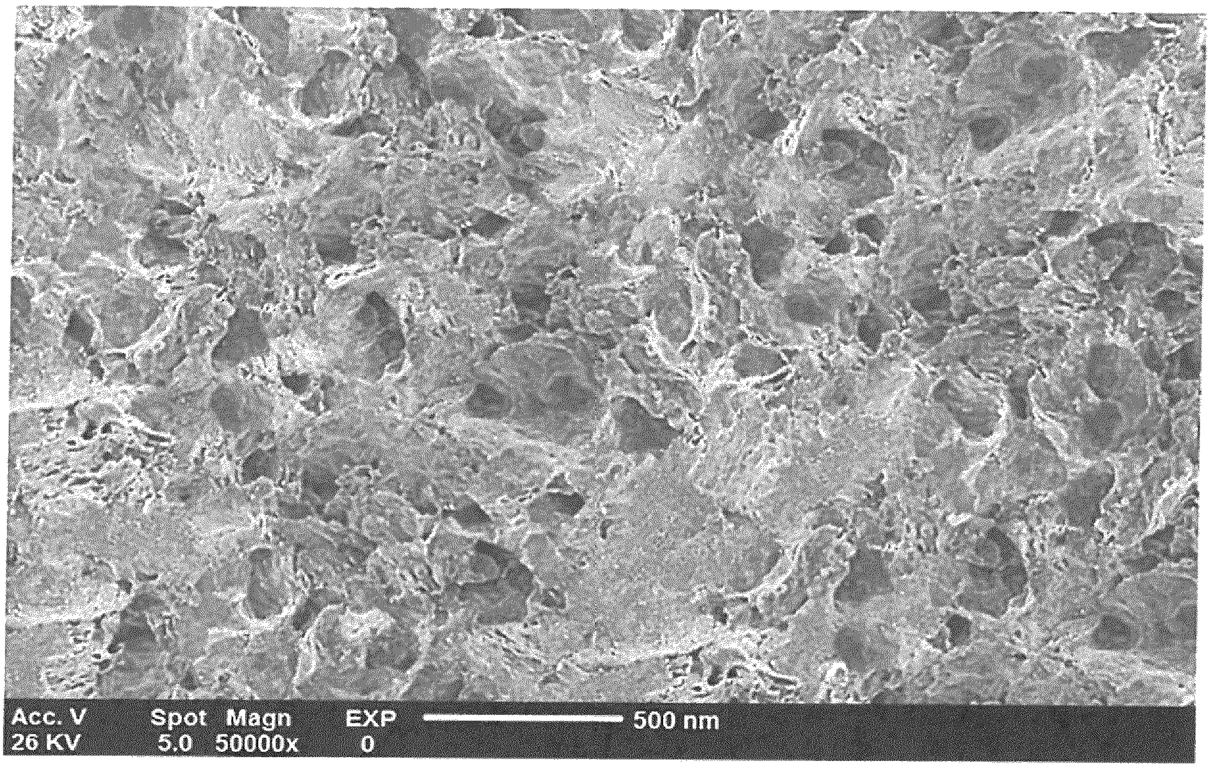
Acc. V
26 KV
Spot
5.0
Magn
50000x
EXP
0
500 nm

Fig. 39

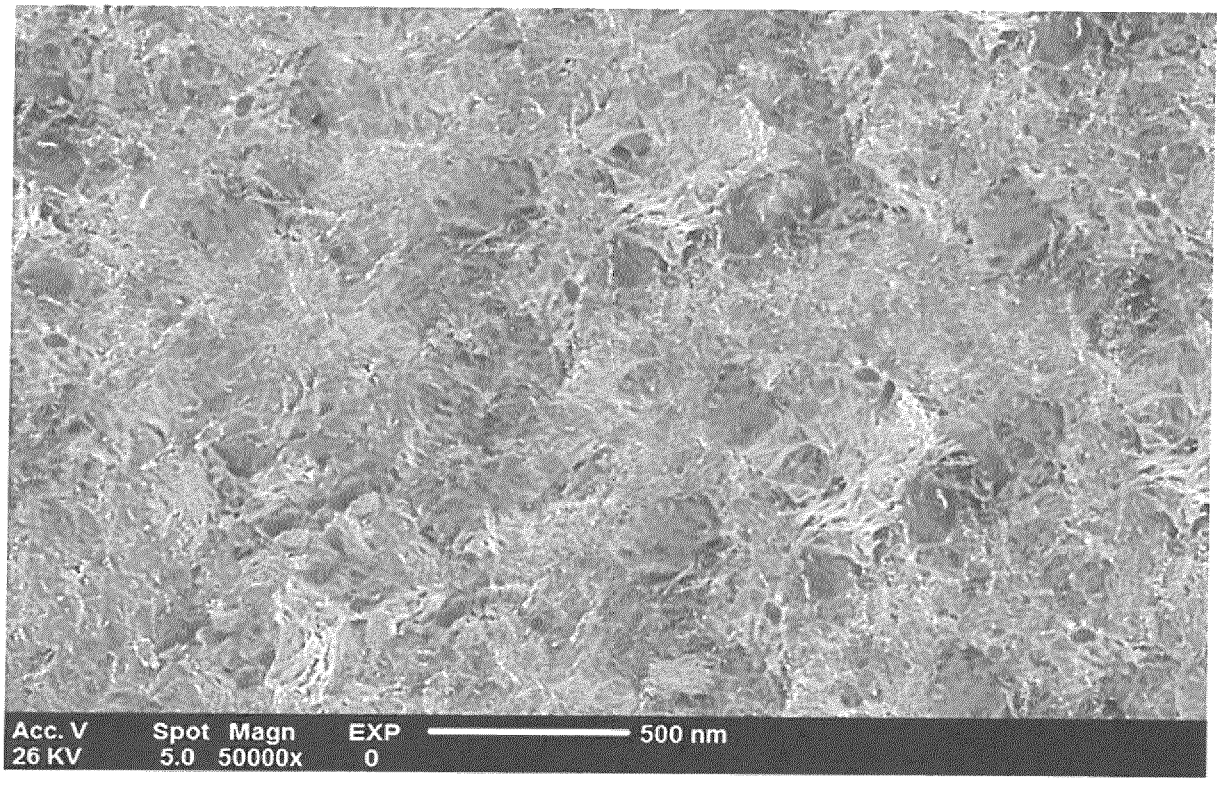
Acc. V
26 KV
Spot
5.0
Magn
50000x
EXP
0
500 nm

Fig. 40

Europäisches Patentamt
European Patent Office
Office européen des brevets

# EUROPEAN SEARCH REPORT

Application Number
EP 24 20 9729

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | US 2016/376382 A1 (KARLSSON MORGAN [SE] ET AL) 29 December 2016 (2016-12-29)<br>* paragraphs [0001], [0115], [0163]; claim 1; examples 1, 6, 9 *<br>----- | 1-11 | INV.<br>C08B37/08<br>A61K8/73<br>C08J3/075<br>C08J3/24 |

TECHNICAL FIELDS SEARCHED (IPC)

C08B
A61Q
C08J
A61K

The present search report has been drawn up for all claims

1

| Place of search | Date of completion of the search | Examiner |
|---|---|---|
| Munich | 24 March 2025 | Arz, Marius |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 24 20 9729

This annex lists the patent family members relating to the patent documents cited in the above-mentioned European search report. The members are as contained in the European Patent Office EDP file on The European Patent Office is in no way liable for these particulars which are merely given for the purpose of information.

24-03-2025

| Patent document cited in search report | Publication date | Patent family member(s) | Publication date |
|---|---|---|---|
| US 2016376382 A1 | 29-12-2016 | AU 2014301493 A1 | 11-02-2016 |
| | | BR 112015031457 A2 | 25-07-2017 |
| | | CA 2916330 A1 | 31-12-2014 |
| | | CN 105473623 A | 06-04-2016 |
| | | CN 110003360 A | 12-07-2019 |
| | | HK 1224315 A1 | 18-08-2017 |
| | | JP 6479783 B2 | 06-03-2019 |
| | | JP 2016523303 A | 08-08-2016 |
| | | KR 20160025026 A | 07-03-2016 |
| | | MX 368941 B | 22-10-2019 |
| | | RU 2016101736 A | 02-08-2017 |
| | | US 2016376382 A1 | 29-12-2016 |
| | | US 2019062461 A1 | 28-02-2019 |
| | | WO 2014206701 A1 | 31-12-2014 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10314946 B, Klein, J., Goldberg, R., luster, N., & Sorkin, R.
- US 9327061 B, Govil, A., Jones, M. L., & Lubock, P.
- US 8647271 B, Müller, A., Herbrechtsmeier, P., Knuth, M., & Nikolaus, K.
- US 10266611 B, De Rosa, M., D'agostino, A., La Gatta, A., & Schiraldi, C.
- US 7196180 B, Aeschlimann, D., & Bulpitt, P.
- US 39659722, Njikang, G. N., Yu, X., Liu, F., & Manesis, N. J.
- US 11260015 B, Lebreton, P., & Guetta, O
- US 10905797 B, Guillen, K. H., & Tezel, A.
- US 8455459 B, Wortzman, M. S., Narins, R., & Lin, X.
- US 8394782 B, Stroumpoulis, D., Mudd, C. S., & Tezel, A.
- US 02234423 A, Tranchepain, F., Brunel, F., Bigand, V., & Vincent, S.
- US 9782490 B, Tauzin, B. V.
- US 9408797 B, Njikang, G. N., Liu, F., Yu, X., Manesis, N. J., & Paliwal, S.
- US 70622113, Njikang, G. N., Yu, X., Liu, F., & Manesis, N. J.
- US 11149319, Karlsson, M., & Edsman, K.
- US 60458722 A, Barral, G.


### Non-patent literature cited in the description


- **SHARMA, S.** ; **TIWARI, S.** A review on bio macromolecular hydrogel classification and its applications.. *International journal of biological macromolecules*, 2020, vol. 162, 737-747
- **SÁNCHEZ-CID, P** ; **JIMENEZ-ROSADO, M.** ; **ROMERO, A.** ; **PÉREZ-PUYANA, V.** Novel trends in hydrogel development for biomedical applications: A review. *Polymers*, 2022, vol. 14 (15), 3023
- **CASCONE, S.** ; **LAMBERTI, G**. Hydrogel-based commercial products for biomedical applications: A review.. *International journal of pharmaceutics*, 2020, vol. 573, 118803
- **BASHIR, S.** ; **HINA, M** ; **IQBAL, J.** ; **RAJPAR, A. H.** ; **MUJTABA, M. A** ; **ALGHAMDI, N. A.** ; **RAMESH, S**. Fundamental concepts of hydrogels: Synthesis, properties, and their applications.. *Polymers*, 2020, vol. 12 (11), 2702
- **YANG, J** ; **CHEN, Y** ; **ZHAO, L.** ; **ZHANG, J** ; **LUO, H.** Constructions and properties of physically cross-linked hydrogels based on natural polymers.. *Polymer Reviews*, 2023, vol. 63 (3), 574-612
- **PITA-LÓPEZ, M. L.** ; **FLETES-VARGAS, G.** ; **ESPINOSA-ANDREWS, H.** ; **RODRIGUEZ-RODRIGUEZ, R.** Physically cross-linked chitosan-based hydrogels for tissue engineering applications: A state-of-the-art review.. *European Polymer Journal*, 2021, vol. 145, 110176
- Cross-linked thermoplastics.. **PATTERSON, R.** ; **KANDELBAUER, A.** ; **MÜLLER, U.** ; **LAMMER, H.** Handbook of thermoset plastics. William Andrew Publishing, 2022, 719-756
- **ALAVARSE, A. C.** ; **FRACHINI, E. C. G.** ; **DA SILVA, R. L. C. G.** ; **LIMA, V. H.** ; **SHAVANDI, A.** ; **PETRI, D. F. S.** Crosslinkers for polysaccharides and proteins: Synthesis conditions, mechanisms, and crosslinking efficiency, a review.. *International journal of biological macromolecules*, 2022, vol. 202, 558-596
- **MUIR, V. G.** ; **BURDICK, J. A.** Chemically modified biopolymers for the formation of biomedical hydrogels. *Chemical reviews*, 2020, vol. 121 (18), 10908-10949
- Preparation methods and structure of hydrogels.. **PEPPAS, N. A.** ; **MIKOS, A. G.** Hydrogels in medicine and pharmacy. CRC press, 2019, 1-26
- **GEORGE, A.** ; **SANJAY, M. R.** ; **SRISUK, R.** ; **PARAMESWARANPILLAI, J.** ; **SIENGCHIN, S. A**. comprehensive review on chemical properties and applications of biopolymers and their composites.. *International journal of biological macromolecules*, 2020, vol. 154, 329-338
- **MORADALI, M. F** ; **REHM, B. H.** Bacterial biopolymers: from pathogenesis to advanced materials.. *Nature Reviews Microbiology*, 2020, vol. 18 (4), 195-210
- **MUIR, V. G.** ; **BURDICK, J. A.** Chemically modified biopolymers for the formation of biomedical hydrogels.. *Chemical reviews*, 2020, vol. 121 (18), 10908-10949
- **DAS, A.** ; **RINGU, T.** ; **GHOSH, S.** ; **PRAMANIK, N.** A comprehensive review on recent advances in preparation, physicochemical characterization, and bioengineering applications of biopolymers.. *Polymer Bulletin*, 2023, vol. 80 (7), 7247-7312

- **NARINS, R. S.** ; **BOWMAN, P. H.** Injectable skin fillers.. *Clinics in plastic surgery*, 2005, vol. 32 (2), 151-162
- **CALABRO, A.** ; **OKEN, M. M.** ; **HASCALL, V. C.** ; **MASELLIS, A. M.** Characterization of hyaluronan synthase expression and hyaluronan synthesis in bone marrow mesenchymal progenitor cells: predominant expression of HAS1 mRNA and up-regulated hyaluronan synthesis in bone marrow cells derived from multiple myeloma patients.. *Blood, The Journal of the American Society of Hematology*, 2002, vol. 100 (7), 2578-2585
- **FRASER, J. R. E.** ; **LAURENT, T. C.** ; **LAURENT, U. B. G.** Hyaluronan: its nature, distribution, functions and turnover. *Journal of internal medicine*, 1997, vol. 242 (1), 27-33
- **TSANAKTSIDOU, E.** ; **KAMMONA, O.** ; **KIPARISSIDES, C.** Recent developments in hyaluronic acid-based hydrogels for cartilage tissue engineering applications.. *Polymers*, 2022, vol. 14 (4), 839
- **YANG, H.** ; **SONG, L.** ; **ZOU, Y.** ; **SUN, D.** ; **WANG, L** ; **YU, Z.** ; **GUO, J.** Role of hyaluronic acids and potential as regenerative biomaterials in wound healing.. *ACS Applied Bio Materials*, 2020, vol. 4 (1), 311-324
- **WATER, J. J** ; **SCHACK, M. M.** ; **VELAZQUEZ-CAMPOY, A.** ; **MALTESEN, M. J** ; **VAN DE WEERT, M.** ; **JORGENSEN, L.** Complex coacervates of hyaluronic acid and lysozyme: Effect on protein structure and physical stability.. *European Journal of Pharmaceutics and Biopharmaceutics*, 2014, vol. 88 (2), 325-331
- **EDSMAN, K.** ; **NORD, L. I.** ; **OHRLUND, Å.** ; **LÄRKNER, H.** ; **KENNE, A. H.** Gel properties of hyaluronic acid dermal fillers.. *Dermatologic Surgery*, 2012, vol. 38 (7), 1170-1179
- **SCHANTÉ, C. E.** ; **ZUBER, G.** ; **HERLIN, C.** ; **VARIDAMME, T. F.** Chemical modifications of hyaluronic acid for the synthesis of derivatives for a broad range of biomedical applications.. *Carbohydrate polymers*, 2011, vol. 85 (3), 469-489
- **TOMIHATA, K.** ; **IKADA, Y.** Preparation of cross-linked hyaluronic acid films of low water content.. *Biomaterials*, 1997, vol. 18 (3), 189-195
- **WOODWARD, J.** ; **RANJIT-REEVES, R.** ; **KATZ, D. F.** ; **BERNARDINI, F. P.** ; **FAGIEN, S.** Comparing Water Absorption of Food and Drug Administration-Approved Hyaluronic Acid Fillers.. *Dermatologic Surgery*, 2021, vol. 47 (9), 1237-1242
- **SHOPE, C.** ; **ANDREWS, L.** ; **EASON, C.** ; **SCHLESINGER, T.** Juvéderm: What we know now.. *Dermatological Reviews*, 2023, vol. 4 (2), 90-95
- **RAMLI, H.** ; **ZAINAL, N. F. A.** ; **HESS, M.** ; **CHAN, C. H.** Basic principle and good practices of rheology for polymers for teachers and beginners.. *Chemistry Teacher International*, 2022, vol. 4 (4), 307-326
- **EBARA, M.** ; **KOTSUCHIBASHI, Y.** ; **NARAIN, R.** ; **IDOTA, N.** ; **KIM, Y. J.** ; **HOFFMAN, J. M.** ; **AOYAGI, T.** Smart biomaterials. Springer, 2014